(19)

**Europäisches Patentamt**
**European Patent Office**
**Office européen des brevets**

(11) **EP 4 397 975 A1**

(12) **EUROPEAN PATENT APPLICATION**
published in accordance with Art. 153(4) EPC

(43) Date of publication:
**10.07.2024 Bulletin 2024/28**

(21) Application number: **22864507.3**

(22) Date of filing: **29.08.2022**

(51) International Patent Classification (IPC):
*G01N 33/573* (2006.01)          *C07K 16/40* (2006.01)
*C12Q 1/68* (2018.01)          *C12Q 1/6876* (2018.01)

(52) Cooperative Patent Classification (CPC):
**C07K 16/40; C12Q 1/68; C12Q 1/6876;**
**G01N 33/573**

(86) International application number:
**PCT/JP2022/032442**

(87) International publication number:
**WO 2023/032925 (09.03.2023 Gazette 2023/10)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **30.08.2021 JP 2021140023**

(71) Applicants:
• **Kyoto University**
  **Kyoto-shi, Kyoto 606-8501 (JP)**
• **Sanyo Chemical Industries, Ltd.**
  **Kyoto-shi, Kyoto 605-0995 (JP)**

(72) Inventors:
• **MOGAMI, Haruta**
  **Kyoto-shi, Kyoto 606-8501 (JP)**

• **KONDOH, Eiji**
  **Kyoto-shi, Kyoto 606-8501 (JP)**
• **UEDA, Yusuke**
  **Kyoto-shi, Kyoto 606-8501 (JP)**
• **TOMONO, Yuki**
  **Kyoto-shi, Kyoto 605-0995 (JP)**
• **IWASAWA, Daijiro**
  **Kyoto-shi, Kyoto 605-0995 (JP)**
• **KUROKAWA, Masato**
  **Kyoto-shi, Kyoto 605-0995 (JP)**

(74) Representative: **Mewburn Ellis LLP**
**Aurora Building**
**Counterslip**
**Bristol BS1 6BX (GB)**

Remarks:
The complete document including Reference
Table(s) and the Sequence Listing(s) can be
downloaded from the EPO website

(54) **TESTING METHOD AND TESTING REAGENT**

(57)    Provided is a testing method using a biomarker for prediction of success or failure of induction of labor, prediction of preterm birth, and prediction of preterm labor, which can be performed with high reliability, simply, and non-invasively. The testing method of the present invention is a testing method using an expression level of 15-PGDH as an index for success or failure of induction of labor, the testing method including a step of detecting the expression level of 15-PGDH in a specimen, the specimen being a vaginal secretion.

FIG.3A

mRNA of 15-PGDH in vaginal secretion

EP 4 397 975 A1

**Description**

TECHNICAL FIELD

The present invention relates to a testing method and a testing reagent.

BACKGROUND ART

A case where continuation of pregnancy is dangerous to a mother and a fetus, such as premature rupture of membrane, pregnancy-induced hypertension, medical complications of the mother, and post-term pregnancy, occurs in about 13 to 150 of a total number of deliveries.
In such a case, induction of labor is indicated to terminate pregnancy.
However, if labor is induced in a state in which a cervix is not ripened, 1) an opening of uterus may not be opened and thus the fetus may not be born (that is, failure of induction), or 2) unnecessary stress may be applied to the fetus due to an excessive increase in intrauterine pressure due to uterine contraction, or uterine rupture rarely occurs, and the mother and the fetus may be put at risk of life.
Currently, regarding whether or not to perform induction of labor, a method for subjectively evaluating a Bishop score (cervical effacement, cervical consistency, opening, cervical position, station) by internal examination (Non Patent Literatures 1 and 2) is known.
A preterm birth rate in Japan is about 5 to 6% (the number of births in 2019 is 860,000, and the number of preterm births is about 50,000) of a total number of deliveries, and accounts for most of neonatal deaths. In the case of preterm birth, these neonates may be left with disabilities.
For example, a postnatal risk of hypertension, diabetes, dyslipidemia, developmental disorders, or schizophrenia would be increased in adulthood by preterm birth.
There is a method for determining the prediction of preterm birth by quantifying fetal fibronectin and granulocyte elastase using an extracellular matrix of fetal membranes as a specimen (Non Patent Literatures 3 and 4).

CITATION LIST

- Non Patent Literature

     Non Patent Literature 1: Obstetrics and Gynecology, 1964, 24(2), p. 266-268
     Non Patent Literature 2: Obstetrics and Gynecology, 2006, 107(2), p. 227-233
     Non Patent Literature 3: The New England Journal of Medicine, 1991, 325(10), p. 669-674
     Non Patent Literature 4: Acta Obsret Cynecol Scand, 1991, 70(1), p. 29-34

SUMMARY OF INVENTION

- Technical Problem

The prediction of success or failure of induction of labor using a Bishop score described in Non Patent Literatures 1 and 2 has a problem of insufficient accuracy.
The method for determining the prediction of preterm birth by quantifying fetal fibronectin and granulocyte elastase described in Non Patent Literatures 3 and 4 has insufficient reliability and difficulty in early prediction.
The present invention has been made to solve the above problems, and an object of the present invention is to provide a testing method using a biomarker for prediction of success or failure of induction of labor, prediction of preterm birth, and prediction of preterm labor, which can be performed with high reliability, simply, and non-invasively.
Furthermore, an object of the present invention is to provide a testing reagent for prediction of success or failure of induction of labor, a testing reagent for prediction of preterm birth, and a testing reagent for prediction of preterm labor.

- Solution to Problem

As a result of intensive studies to achieve the above object, the present inventors have found that an expression level of 15-PGDH in a vaginal secretion is an index for success or failure of induction of labor, an index for preterm birth, and an index for preterm labor, and have reached the present invention.
That is, the present invention relates to a testing method using an expression level of 15-PGDH as an index for success or failure of induction of labor, the testing method including a step of detecting the expression level of 15-PGDH in a specimen, the specimen being a vaginal secretion; a testing method using an expression level of 15-PGDH as an index for preterm birth, the testing method including a step of detecting the expression level of 15-PGDH in a specimen, the specimen being a vaginal secretion; a testing method using an expression level of 15-PGDH as an index for preterm labor, the testing method including a step of detecting the expression level of 15-PGDH in a specimen, the specimen being a vaginal secretion; a testing reagent for prediction of success or failure of induction of labor, the testing reagent including an anti-15-PGDH antibody or a primer for 15-PGDH; a testing reagent for prediction of preterm birth, the testing reagent including an anti-15-PGDH antibody or a primer for 15-PGDH; and a testing reagent for prediction of preterm labor, the testing reagent including an anti-15-PGDH antibody or a primer for 15-PGDH.

- Advantageous Effects of Invention

An expression level of 15-PGDH measured using a vaginal secretion as a specimen is an index for success or failure of induction of labor, an index for preterm birth, and an index for preterm labor.

Therefore, according to the present invention, it is possible to provide a testing method using a biomarker for success or failure of induction of labor, preterm birth, and preterm labor, which can be performed with high reliability, simply, and non-invasively.

Furthermore, it is possible to provide a testing reagent for prediction of success or failure of induction of labor, a testing reagent for prediction of preterm birth, and a testing reagent for prediction of preterm labor.

BRIEF DESCRIPTION OF DRAWINGS

FIG. 1A is a photograph after immunohistostaining of a cervical tissue using an anti-15-PGDH antibody.

FIG. 1B is a photograph after immunohistostaining of a cervical tissue using an anti-15-PGDH antibody.

FIG. 2 is a photograph of a PVDF membrane when Western blot is performed in a test for confirming an expression site of 15-PGDH.

FIG. 3A is a diagram showing a distribution of values of relative fold expression using quantitative values of mRNA of 15-PGDH in a labor-induction success group and a labor-induction failure group.

FIG. 3B is a diagram showing a distribution of values of relative fold expression using quantitative values of mRNA of 15-PGDH in a labor-induction success group (excluding subject 1-7) and a labor-induction failure group.

FIG. 4A is a diagram showing a distribution of values of relative fold expression using quantitative values of mRNA of 15-PGDH in a labor-induction success group and a labor-induction failure group in subjects of only primiparous women.

FIG. 4B is a diagram showing a distribution of values of relative fold expression using quantitative values of mRNA of 15-PGDH in a labor-induction success group (excluding subject 1-7) and a labor-induction failure group in subjects of only primiparous women.

FIG. 5 is a diagram showing a distribution of values of relative fold expression using quantitative values of 15-PGDH in a labor-induction success group and a labor-induction failure group.

FIG. 6A is a diagram showing a distribution of values of Bishop score in a labor-induction success group and a labor-induction failure group.

FIG. 6B is a diagram showing a distribution of values of Bishop score in a labor-induction success group (excluding subject 1-7) and a labor-induction failure group.

FIG. 7A is a diagram showing a distribution of values of Bishop score in a labor-induction success group and a labor-induction failure group in subjects of only primiparous women.

FIG. 7B is a diagram showing a distribution of values of Bishop score in a labor-induction success group (excluding subject 1-7) and a labor-induction failure group in subjects of only primiparous women.

FIG. 8 is a diagram showing a distribution of quantitative values of 15-PGDH in a labor-induction success group and a labor-induction failure group.

FIG. 9 is a diagram showing a distribution of quantitative values of 15-PGDH in a labor-induction success group and a labor-induction failure group in subjects of only primiparous women.

FIG. 10 is a diagram showing a distribution of 15-PGDH correction values in a labor-induction success group and a labor-induction failure group.

FIG. 11 is a diagram showing a distribution of 15-PGDH correction values in a labor-induction success group and a labor-induction failure group in subjects of only primiparous women.

DESCRIPTION OF EMBODIMENTS

(First embodiment)

A testing method according to a first embodiment of the present invention is a testing method using an expression level of 15-PGDH as an index for success or failure of induction of labor, the testing method including a step of detecting the expression level of 15-PGDH in a specimen, the specimen being a vaginal secretion.
In the testing method according to the first embodiment of the present invention, a vaginal secretion is used as a specimen.
Therefore, the testing method is non-invasive and less burdensome to a pregnant woman.
The testing method according to the first embodiment of the present invention includes a step of detecting an expression level of 15-PGDH in a vaginal secretion.
The expression level of 15-PGDH in a vaginal secretion can be an index for success or failure of induction of labor.
Therefore, by using the testing method according to the first embodiment of the present invention, it is possible to predict success or failure of induction of labor with high reliability and simply.
A reason why the expression level of 15-PGDH is an index for success or failure of induction of labor is considered to be due to the following action.
15-PGDH functions as a prostaglandin-inactivating enzyme. Therefore, since prostaglandin has a function of promoting delivery, 15-PGDH acts suppressively on delivery.
Therefore, when the expression level of 15-PGDH is high in a vagina in a pregnant woman, induction of labor is predicted to be unlikely to be successful in the pregnant woman.
In the testing method according to the first embodiment of the present invention, an index based on a value obtained by dividing the expression level of 15-PGDH by an expression level of prostaglandin may be used as an index for success or failure of induction of labor.
In the testing method according to the first embodiment of the present invention, when induction of labor is determined to be unlikely to be successful, induction of labor is not performed and preparation can be performed for terminating pregnancy in advance by another method such as cesarean section. Therefore, it is possible to avoid putting a mother and a fetus at risk of life.
In the testing method according to the first embodiment of the present invention, the vaginal secretion is preferably a secretion from an epithelium of a cervix and/or a vaginal squamous epithelium.
By using these vaginal secretions, it is possible to predict success or failure of induction of labor with higher reliability.
In the testing method according to the first embodiment of the present invention, the vaginal secretion is preferably a secretion derived from a primiparous woman.
When the vaginal secretion is a secretion derived from a primiparous woman, it is possible to predict success or failure of induction of labor with higher reliability.
In the testing method according to the first embodiment of the present invention, the detection of 15-PGDH may be detection by immunological measurement or detection by gene-related testing.
Each detection is described in detail below.

(Immunological measurement)

In the testing method according to the first embodiment of the present invention, when 15-PGDH is measured by immunological measurement, the 15-PGDH can be detected sensitively, accurately, quickly, and simply.
The testing method according to the first embodiment of the present invention preferably includes a step of quantifying the 15-PGDH.
By quantifying the 15-PGDH, it is possible to predict success or failure of induction of labor with higher reliability.
For quantification by immunological measurement, a monoclonal antibody and/or a polyclonal antibody against 15-PGDH (hereinafter, when it is not necessary to distinguish these antibodies, these antibodies are also collectively and simply referred to as "anti-15-PGDH antibody") can be used.
A monoclonal antibody is preferably used from the viewpoint of favorable specificity to 15-PGDH.
In the testing method according to the first embodiment of the present invention, a monoclonal antibody against 15-PGDH produced by a conventionally known method can be used. For example, a monoclonal antibody produced by a known method using, for example, a cell fusion technique or a gene recombination technique [Eur. J immunol, 6, 511 (1976)] can be used.
Specific examples of the polyclonal antibody against 15-PGDH include NB200-179 manufactured by Novus Biologicals and the like. Specific examples of the monoclonal antibody include 15-PGDH/HPGD Rabbit mAb (product number: A5024) manufactured by ABclonal, Inc. and the like.

In the testing method according to the first embodiment of the present invention, examples of the immunological measurement method include radioimmunoassay (RIA), fluorescence immunoassay (FIA), enzyme immunoassay (EIA), electrochemiluminescence immunoassay (ECLIA), chemiluminescence immunoassay (CLIA), turbidimetric immnoassay (TIA), and latex agglutination turbidimetric immunoassay (LTIA). Among them, from the viewpoint of measurement sensitivity, fluorescence immunoassay (FIA), enzyme immunoassay (EIA), electrochemiluminescence immunoassay (ECLIA), and chemiluminescence immunoassay (CLIA) are more preferable, and enzyme immunoassay (EIA) is still more preferable. Among enzyme immunoassays (EIAs), chemiluminescence enzyme immunoassay (CLEIA) using a chemiluminescent substance as an enzyme substrate is particularly preferable.

In the testing method according to the first embodiment of the present invention, when 15-PGDH in a specimen is quantified by enzyme immunoassay (EIA), 15-PGDH and/or an analog thereof to be introduced from the outside, an anti-15-PGDH antibody, or a secondary antibody is labeled for chemical color development and luminescence.

The label is not limited, but is preferably alkaline phosphatase, $\beta$-galactosidase, peroxidase (POD), microperoxidase, glucose oxidase, glucose-6-phosphate dehydrogenase, malate dehydrogenase, luciferase, tyrosinase, or acid phosphatase.

From the viewpoint of detection sensitivity and the like, alkaline phosphatase, peroxidase, or glucose oxidase is more preferably used as a label used for chemical color development/luminescence, and peroxidase is still more preferably used.

In the testing method according to the first embodiment of the present invention, when 15-PGDH is quantified by immunological measurement, for example, an anti-15-PGDH antibody, 15-PGDH, and/or an analog thereof is preferably supported on a solid-phase carrier, and 15-PGDH is preferably quantified using the solid-phase carrier.

The solid-phase carrier used in the testing method according to the first embodiment of the present invention is not limited, and examples thereof include glass beads, polystyrene beads, magnetic particles such as magnetic silica particles, microplates, and latex. Among them, glass beads and/or magnetic silica particles are preferably used from the viewpoint of detection sensitivity and the like.

In the testing method according to the first embodiment of the present invention, as the immunological measurement, for example, the measurement described in Enzyme Immunoassay (published on May 15, 1987, 3rd edition, Igaku-Shoin Ltd.) or the like can be used. Non-competitive immunological measurement or competitive immunological measurement can be used as the immunological measurement.

In the present description, the non-competitive immunological measurement means measurement including a step of binding 15-PGDH in a specimen to an anti-15-PGDH antibody without adding 15-PGDH and/or an analog thereof from the outside and without causing competition.

In the present description, the competitive immunological measurement means measurement including a step of adding 15-PGDH and/or an analog thereof from the outside, and causing 15-PGDH in a specimen and the 15-PGDH and/or the analog thereof introduced from the outside to compete with each other to bind them to an anti-15-PGDH antibody.

Hereinafter, a first non-competitive immunological measurement, which is an example of the testing method for quantifying 15-PGDH in a specimen by non-competitive immunological measurement according to the testing method of the first embodiment of the present invention, is described.

(1) Step of preparing testing material

In this step, a solid-phase carrier carrying an anti-15-PGDH antibody, a buffer for immune reaction, and a labeled anti-15-PGDH antibody, which are materials used for testing, are prepared.

Production of the solid-phase carrier carrying an anti-15-PGDH antibody is described.

A method for supporting an anti-15-PGDH antibody on a solid-phase carrier is not limited, and a conventional method can be adopted.

The solid-phase carrier is not limited, and examples thereof include glass beads, polystyrene beads, magnetic particles such as magnetic silica particles, microplates, and latex. Among them, glass beads and/or magnetic silica particles are preferably used from the viewpoint of detection sensitivity and the like.

As the glass beads and/or magnetic silica particles, for example, magnetic silica particles described in JP 2013-19889 A and the like, commercially available Dynabeads (manufactured by Thermo Fisher Scientific, Inc.), and the like can be used.

Next, the buffer for immune reaction is described.

The buffer for immune reaction is preferably a buffer having a buffering action at pH 5.0 to 10.0, more preferably a buffer having a buffering action at pH 6.0 to 9.0.

Examples of such a buffer for immune reaction include a phosphate buffer, a Tris buffer, a Good buffer, a glycine buffer, and a borate buffer.

In such a buffer, for example, a stabilizer (e.g., albumin, globulin, water-soluble gelatin, and polyethylene glycol), a surfactant, and a saccharide may be contained as long as the target immune reaction is not inhibited.

Next, production of the labeled anti-15-PGDH antibody is described.

A label attached to an anti-15-PGDH antibody may be a label of a radioisotope, or may be a label that chemically develops color and emits light. Among them, from the viewpoint of safety, a label that chemically develops color and emits light is preferable.

These labels can be attached to an anti-15-PGDH antibody by a conventional method.

(2) Step of creating calibration curve

(2-1) Step of producing 15-PGDH standard solution

15-PGDH is added to the buffer for immune reaction to produce a plurality of 15-PGDH standard solutions having different concentrations.

(2-2) Step of forming anti-15-PGDH antibody-15-PGDH complex supported on solid-phase carrier

First, 15-PGDH in each 15-PGDH standard solution is reacted with an anti-15-PGDH antibody supported on a solid-phase carrier. At this time, the buffer for immune reaction is used as a buffer for the reaction system.

By this reaction, an (anti-15-PGDH antibody)-(15-PGDH) complex supported on a solid-phase carrier can be formed.

Thereafter, unreacted 15-PGDH is removed from the reaction system.

(2-3) Step of forming (anti-15-PGDH antibody)-(15-PGDH)-(labeled anti-15-PGDH antibody) complex supported on solid-phase carrier

Next, a labeled anti-15-PGDH antibody is added to the reaction system, and the anti-15-PGDH antibody-15-PGDH complex supported on a solid-phase carrier is reacted with the labeled anti-15-PGDH antibody.

By this reaction, an (anti-15-PGDH antibody)-(15-PGDH)-(labeled anti-15-PGDH antibody) complex supported on a solid-phase carrier can be formed.

Thereafter, an unreacted labeled anti-15-PGDH antibody is removed from the reaction system.

(2-4) Step of counting label

Next, the label of the (anti-15-PGDH antibody)-(15-PGDH)-(labeled anti-15-PGDH antibody) complex supported on a solid-phase carrier remaining in the reaction system is counted.

The label can be counted by a conventional method according to the type of the label.

(2-5) Step of creating calibration curve

A calibration curve showing a relationship between a concentration of 15-PGDH and the number of counts of labels is created based on a concentration of each 15-PGDH standard solution and the number of counts of labels obtained.

(3) Step of forming (anti-15-PGDH antibody)-(15-PGDH) complex supported on solid-phase carrier

The buffer for immune reaction is used as a buffer for the reaction system, and 15-PGDH in a specimen is reacted with an anti-15-PGDH antibody supported on a solid-phase carrier.

By this reaction, an (anti-15-PGDH antibody)-(15-PGDH) complex supported on a solid-phase carrier can be formed.

Thereafter, unreacted 15-PGDH is removed from the reaction system.

(4) Step of forming (anti-15-PGDH antibody)-(15-PGDH)-(labeled anti-15-PGDH antibody) complex supported on solid-phase carrier

Next, a labeled anti-15-PGDH antibody is added to the reaction system, and the (anti-15-PGDH antibody)-(15-PGDH) complex supported on a solid-phase carrier is reacted with the labeled anti-15-PGDH antibody.

By this reaction, an (anti-15-PGDH antibody)-(15-PGDH)-(labeled anti-15-PGDH antibody) complex supported on a solid-phase carrier can be formed.

Thereafter, an unreacted labeled anti-15-PGDH antibody is removed from the reaction system.

(5) Step of counting label

Next, the label of the (anti-15-PGDH antibody)-(15-PGDH)-(labeled anti-15-PGDH antibody) complex supported on a solid-phase carrier remaining in the reaction system is counted.
The label can be counted by a conventional method according to the type of the label.

(6) Quantification step

Next, a concentration of 15-PGDH in the specimen is calculated based on the obtained number of counts and the created calibration curve.
Through the above steps, 15-PGDH in the specimen can be quantified.
In the testing method according to the first embodiment of the present invention, in the non-competitive immunological measurement, measurement may be performed using a labeled secondary antibody without using a labeled anti-15-PGDH antibody.
Hereinafter, a second non-competitive immunological measurement, which is an example of the testing method for quantifying 15-PGDH in a specimen by non-competitive immunological measurement according to the testing method of the present invention, is described.

(1) Step of preparing testing material

In this step, a solid-phase carrier for carrying a first anti-15-PGDH antibody, a buffer for immune reaction, a first anti-15-PGDH antibody to which a secondary antibody cannot be bound, a second anti-15-PGDH antibody to which a secondary antibody can be bound, and a labeled secondary antibody, which are materials used for testing, are prepared.
First, a first anti-15-PGDH antibody is supported on a solid-phase carrier. As the type of the solid-phase carrier, the same solid-phase carrier as described in the first non-competitive immunological measurement can be used.
A secondary antibody is labeled to produce a labeled secondary antibody.
The label attached to the secondary antibody may be a label of a radioisotope, or may be a label that chemically develops color and emits light. Among them, from the viewpoint of safety, a label that chemically develops color and emits light is preferable.
These labels can be attached to a secondary antibody by a conventional method.
As the buffer for immune reaction, the same buffer for immune reaction as described in the first non-competitive immunological measurement can be used.

(2) Step of creating calibration curve

(2-1) Step of producing 15-PGDH standard solution

15-PGDH is added to the buffer for immune reaction to produce a plurality of 15-PGDH standard solutions having different concentrations.

(2-2) Step of forming (first anti-15-PGDH antibody)-(15-PGDH) complex

15-PGDH in each 15-PGDH standard solution is reacted with a first anti-15-PGDH antibody supported on a solid-phase carrier. At this time, the buffer for immune reaction is used as a buffer for the reaction system.
By this reaction, a (first anti-15-PGDH antibody)-(15-PGDH) complex can be formed.
Thereafter, unreacted 15-PGDH is removed from the reaction system.

(2-3) Step of forming (first anti-15-PGDH antibody)-(15-PGDH)-(second anti-15-PGDH antibody) complex

Next, a second anti-15-PGDH antibody is added to the reaction system, and the (first anti-15-PGDH antibody)-(15-PGDH) complex is reacted with the second anti-15-PGDH antibody.
By this reaction, a (first anti-15-PGDH antibody)-(15-PGDH)-(second anti-15-PGDH antibody) complex can be formed.
Next, an unreacted second anti-15-PGDH antibody is removed from the reaction system.

(2-4) Step of binding labeled secondary antibody

Next, a labeled secondary antibody is added to the reaction system, and the (first anti-15-PGDH antibody)-(15-PGDH)-(second anti-15-PGDH antibody) complex is reacted with the labeled secondary antibody.
Thereby, a (first anti-15-PGDH antibody)-(15-PGDH)-(second anti-15-PGDH antibody)-(labeled antibody) complex can be formed.
Next, an unreacted labeled secondary antibody is removed from the reaction system.

(2-5) Step of counting label

Next, the label of the (first anti-15-PGDH antibody)-(15-PGDH)-(second anti-15-PGDH antibody)-(labeled secondary antibody) complex remaining in the reaction system is counted.
The label can be counted by a conventional method according to the type of the label.

(2-6) Step of creating calibration curve

A calibration curve showing a relationship between a concentration of 15-PGDH and the number of counts of labels is created based on a concentration of each 15-PGDH standard solution and the number of counts of labels obtained.

(3) Step of forming (first anti-15-PGDH antibody)-(15-PGDH) complex

The buffer for immune reaction is used as a buffer for the reaction system, and 15-PGDH in a specimen is reacted with a first anti-15-PGDH antibody supported on a solid-phase carrier.
By this reaction, a (first anti-15-PGDH antibody)-(15-PGDH) complex can be formed.
Thereafter, unreacted 15-PGDH is removed from the reaction system.

(4) Step of forming (first anti-15-PGDH antibody)-(15-PGDH)-(second anti-15-PGDH antibody) complex

Next, a second anti-15-PGDH antibody is added to the reaction system, and the (first anti-15-PGDH antibody)-(15-PGDH) complex is reacted with the second anti-15-PGDH antibody.
By this reaction, a (first anti-15-PGDH antibody)-(15-PGDH)-(second anti-15-PGDH antibody) complex can be formed.
Thereafter, an unreacted second anti-15-PGDH antibody is removed from the reaction system.

(5) Step of binding labeled secondary antibody

Next, a labeled secondary antibody is added to the reaction system, and the (first anti-15-PGDH antibody)-(15-PGDH)-(second anti-15-PGDH antibody) complex is reacted with the labeled secondary antibody.
Thereby, a (first anti-15-PGDH antibody)-(15-PGDH)-(second anti-15-PGDH antibody)-(labeled secondary antibody) complex can be formed.
Thereafter, an unreacted labeled secondary antibody is removed from the reaction system.

(6) Step of counting label

Next, the label of the (first anti-15-PGDH antibody)-(15-PGDH)-(second anti-15-PGDH antibody)-(labeled secondary antibody) complex remaining in the reaction system is counted.
The label can be counted by a conventional method according to the type of the label.

(7) Quantification step

Next, a concentration of 15-PGDH in the specimen is calculated based on the obtained number of counts and the created calibration curve.
Through the above steps, 15-PGDH in the specimen can be quantified.
Next, an example of the testing method for quantifying 15-PGDH in a specimen by competitive immunological measurement according to the testing method of the present invention is described.
In the following description of competitive immunological measurement, for convenience, 15-PGDH to which no label is attached is described as "unlabeled 15-PGDH", and 15-PGDH to which a label is attached is described as "labeled 15-PGDH".

(1) Step of preparing testing material

In this step, a solid-phase carrier carrying an anti-15-PGDH antibody, a buffer for immune reaction, and a labeled 15-PGDH, which are materials used for testing, are prepared.

As the solid-phase carrier carrying an anti-15-PGDH antibody and the buffer for immune reaction, those shown in the description of the first non-competitive immunological measurement can be used.

The labeled 15-PGDH is described.

The labeled 15-PGDH can be produced by labeling unlabeled 15-PGDH.

The label may be a label of a radioisotope, or may be a label that chemically develops color and emits light. Among them, from the viewpoint of safety, a label that chemically develops color and emits light is preferable.

These labels can be attached to unlabeled 15-PGDH by a conventional method.

As described above, an analog of 15-PGDH may be used instead of 15-PGDH.

In this case, a labeled analog of 15-PGDH is used instead of labeled 15-PGDH.

(2) Step of creating calibration curve

(2-1) Step of producing unlabeled 15-PGDH standard solution

Unlabeled 15-PGDH is added to the buffer for immune reaction to produce a plurality of unlabeled 15-PGDH standard solutions having different concentrations.

(2-2) Step of preparing (unlabeled 15-PGDH)-(labeled-15-PGDH) mixed solution

First, a certain amount of labeled 15-PGDH is added to each unlabeled 15-PGDH standard solution and mixed to prepare an (unlabeled 15-PGDH)-(labeled 15-PGDH) mixed solution.

(2-3) Step of forming (anti-15-PGDH antibody)-(15-PGDH) complex

Next, unlabeled 15-PGDH and labeled 15-PGDH in the (unlabeled 15-PGDH)-(labeled 15-PGDH) mixed solution are reacted with an anti-15-PGDH antibody supported on a solid-phase carrier. At this time, unlabeled 15-PGDH and labeled 15-PGDH compete with each other and bind to the anti-15-PGDH antibody.

By this reaction, an (anti-15-PGDH antibody)-(unlabeled 15-PGDH) complex and an (anti-15-PGDH antibody)-(labeled 15-PGDH) complex can be formed.

In this reaction, when an amount of unlabeled 15-PGDH in the (unlabeled 15-PGDH)-(labeled 15-PGDH) mixed solution is large, an amount of the (anti-15-PGDH antibody)-(labeled 15-PGDH) complex is small.

When the amount of unlabeled 15-PGDH in the (unlabeled 15-PGDH)-(labeled-15-PGDH) mixed solution is small, the amount of the (anti-15-PGDH antibody)-(labeled 15-PGDH) complex is large.

Thereafter, unreacted unlabeled 15-PGDH and labeled 15-PGDH are removed.

(2-4) Step of counting label

Next, the label of the (anti-15-PGDH antibody)-(labeled 15-PGDH) complex remaining in the reaction system is counted.

The label can be counted by a conventional method according to the type of the label.

(2-5) Step of creating calibration curve

A calibration curve showing a relationship between a concentration of unlabeled 15-PGDH and the number of counts of labels is created based on a concentration of each unlabeled 15-PGDH standard solution and the number of counts of labels obtained.

(3) Step of preparing (unlabeled 15-PGDH)-(labeled 15-PGDH) mixed solution

Next, a specimen and a certain amount of labeled 15-PGDH are added to the buffer for immune reaction and mixed to prepare a (specimen)-(labeled 15-PGDH) mixed solution.

Since no label is attached to 15-PGDH in the specimen, it is described as "unlabeled 15-PGDH" in the following description.

(4) Step of forming (anti-15-PGDH antibody)-(15-PGDH) complex

Next, unlabeled 15-PGDH and labeled 15-PGDH in the (specimen)-(labeled 15-PGDH) mixed solution are reacted with an anti-15-PGDH antibody supported on a solid-phase carrier. At this time, unlabeled 15-PGDH and labeled 15-PGDH compete with each other and bind to the anti-15-PGDH antibody.

By this reaction, an (anti-15-PGDH antibody)-(unlabeled 15-PGDH) complex and an (anti-15-PGDH antibody)-(labeled 15-PGDH) complex can be formed.

Thereafter, unreacted unlabeled 15-PGDH and labeled 15-PGDH are removed.

(5) Step of counting label

Next, the label of the (anti-15-PGDH antibody)-(labeled 15-PGDH) complex remaining in the reaction system is counted.

The label can be counted by a conventional method according to the type of the label.

(6) Quantification step

Next, a concentration of 15-PGDH (that is, unlabeled 15-PGDH) in the specimen is calculated based on the obtained number of counts and the created calibration curve.

Through the above steps, 15-PGDH in the specimen can be quantified.

A quantitative value of 15-PGDH in the specimen is an index for success or failure of induction of labor.

In the detection of the expression level of 15-PGDH by immunological measurement in the testing method according to the first embodiment of the present invention described above, examples of a method for producing a measurement sample from a collected specimen include the following method.

<Method for producing measurement sample>

The collected specimen is added to a buffer containing a protease inhibitor to obtain a measurement sample.

The buffer is not limited.

The buffer is preferably a buffer containing a surfactant.

Examples of the surfactant include nonionic surfactants (e.g., polyoxyethylene nonylphenyl ether, polyoxyethylene alkyl ether (e.g., polyoxyethylene octyl ether), and polyoxyethylene sorbitan aliphatic ester), anionic surfactants (e.g., sodium deoxycholate, sodium dodecyl sulfate, and sodium lauroyl sarcosine), cationic surfactants (e.g., ethyltrimethylammonium bromide), and zwitterionic surfactants (e.g., CHAPS and CHAPSO).

The surfactant may be used alone or in combination of two or more thereof. In particular, a combination of a nonionic surfactant and an anionic surfactant is preferable.

A weight proportion of the surfactant is preferably 0.01 to 10 wt%, more preferably 0.1 to 5 wt%, based on a weight of the buffer.

A pH of the buffer is preferably 5.0 to 10.0, more preferably 6.0 to 9.0.

The buffer may contain a protein (e.g., bovine serum albumin (BSA), casein, and skim milk).

Among the above buffers, for example, a RIPA buffer is preferable from the viewpoint of improving a matching rate with clinical practice (accuracy).

As an index for success or failure of induction of labor, a relative value of the expression level of 15-PGDH based on an amount of total protein in a specimen may be used.

For example, a total protein concentration in the measurement sample can be measured by, for example, BCA assay.

From the viewpoint of improving the accuracy of prediction of success or failure of induction of labor, a value obtained by dividing a quantitative value of 15-PGDH in a specimen by a quantitative value of prostaglandin in the specimen (quantitative value of 15-PGDH/quantitative value of prostaglandin) may be used as an index for success or failure of induction of labor.

Examples of the prostaglandin include PGE2 and PGF2$\alpha$, and PGE2 is preferably quantified.

As a method for quantifying 15-PGDH in a specimen, the above method can be used.

For quantification of prostaglandin in a specimen, for example, a known measurement kit [e.g., DetectX Prostaglandin E2 Multi-Format ELISA Kit (Arbor Assays Inc.)] can be used.

In the detection of the expression level of 15-PGDH by immunological measurement in the testing method according to the first embodiment of the present invention described above, a solid-phase carrier was used, a calibration curve was created, and 15-PGDH was quantified.

However, in the detection of the expression level of 15-PGDH by immunological measurement in the testing method according to the first embodiment of the present invention, a solid-phase carrier may not be used, and a ratio of 15-PGDH to a predetermined weight of total protein in a specimen may be detected.

Such a method is described below.

(1) Specimen treatment

The collected specimen is added to a buffer containing a protease inhibitor to obtain a measurement sample.

The buffer is not limited.

The buffer is preferably a buffer containing a surfactant.

As such a buffer, the buffer described in the above

<Method for producing measurement sample> is preferably used.

(2) Measurement of amount of total protein in measurement sample

A part of the measurement sample is used to measure a total protein concentration in the measurement sample by BCA assay.

(3) SDS-PAGE

Based on the total protein concentration in the measurement sample, a predetermined weight of protein is taken out from the measurement sample, a loading buffer for SDS-PAGE is added, and heating is performed by a normal method to produce a sample for SDS-PAGE.

Next, a sample for SDS-PAGE is applied to an SDS-polyacrylamide gel, and electrophoresis is performed.

(4) Western blot (measurement of number of counts of 15-PGDH)

The SDS-polyacrylamide gel after electrophoresis is transferred to a PVDF membrane, washed, and then subjected to blocking treatment.

Next, an antigen-antibody reaction is performed using an anti-15-PGDH antibody as a primary antibody to form a (15-PGDH)-(anti-15-PGDH antibody) complex on the PVDF membrane.

Next, the PVDF membrane is washed, and a (15-PGDH)-(anti-15-PGDH antibody)-(labeled secondary antibody) complex is formed using a labeled secondary antibody that binds to an anti-15-PGDH antibody as a secondary antibody.

Next, the PVDF membrane is washed, and the label of the 15-PGDH-anti-15-PGDH antibody-labeled secondary antibody complex on the PVDF membrane is counted.

The label can be counted by a conventional method according to the type of the label.

The label is not limited, but is preferably alkaline phosphatase, $\beta$-galactosidase, peroxidase (POD), microperoxidase, glucose oxidase, glucose-6-phosphate dehydrogenase, malate dehydrogenase, luciferase, tyrosinase, or acid phosphatase.

From the viewpoint of detection sensitivity and the like, alkaline phosphatase, peroxidase, or glucose oxidase is more preferably used as a label used for chemical color development/luminescence, and peroxidase is still more preferably used.

(5) Detection step

Next, the number of counts of 15-PGDH with respect to an amount of total protein in a specimen is calculated based on the obtained number of counts.

The number of counts of 15-PGDH with respect to an amount of total protein in a specimen is an index for the success or failure of induction of labor.

From the viewpoint of improving the accuracy of prediction of success or failure of induction of labor, a value obtained by dividing a quantitative value of 15-PGDH in a specimen by a quantitative value of prostaglandin in the specimen (quantitative value of 15-PGDH/quantitative value of prostaglandin) may be used as an index for success or failure of induction of labor.

Examples of the prostaglandin include PGE2 and PGF2$\alpha$, and PGE2 is preferably quantified.

As a method for quantifying 15-PGDH in a specimen, the above method can be used.

For quantification of prostaglandin in a specimen, for example, a known measurement kit [e.g., DetectX Prostaglandin E2 Multi-Format ELISA Kit (Arbor Assays Inc.)] can be used.

A testing reagent to predict success or failure of induction of labor (that is, a testing reagent for prediction of success or failure of induction of labor) containing an anti-15-PGDH antibody as described above, is the testing reagent of the present invention.

(Gene-related testing)

In the testing method according to the first embodiment of the present invention, by measuring 15-PGDH by gene-related testing, the 15-PGDH can be detected sensitively, accurately, quickly, and simply.

Examples of a method for measuring 15-PGDH by gene-related testing include a PCR method, a Southern blot method, and a Northern blot method.

The testing method according to the first embodiment of the present invention preferably includes a step of quantifying mRNA of the 15-PGDH.

By quantifying mRNA of 15-PGDH (quantifying a signal of an expression level of 15-PGDH as described later), success or failure of induction of labor can be predicted in more detail.

Examples of a method for quantifying mRNA of 15-PGDH by gene-related testing include a quantitative PCR method.

Hereinafter, an example of the method for quantifying mRNA of 15-PGDH by a quantitative PCR method in the testing method according to the first embodiment of the present invention is described.

(1) Extraction of total RNA

First, total RNA is extracted from a specimen.

As a method for extracting total RNA, for example, a specimen is dissolved in RNA later [RNA later Soln (#AM7021, manufactured by Invitrogen)] and centrifuged to obtain a pellet, and total RNA is extracted from the pellet using Sepasol RNA Super G (Nacalai Tesque, Inc., 09379-26).

(2) Production of cDNA library

Thereafter, reverse transcription PCR (RT-PCR) is performed using a predetermined amount of RNA among the extracted total RNA to produce a cDNA library.

When RT-PCR is performed, for example, ReverTra Ace qPCR RT Master Mix (TOYOBO CO., LTD., FSQ-201) can be used.

(3) Quantification of mRNA of 15-PGDH

Next, a reagent for quantitative PCR containing a primer for amplification of 15-PGDH is added to the produced cDNA library, and quantitative PCR is performed, whereby mRNA of 15-PGDH can be quantified.

As the reagent for quantitative PCR, for example, THUNDERBIRD SYBR qPCR Mix (TOYOBO CO., LTD., QPS -201) can be used.

As the primer for amplification of 15-PGDH, a primer having the following Forward sequence and Reverse sequence is preferably used.

Sequence of primer for amplification of 15-PGDH:

Forward sequence: GCCGG TTTAT TGTGC TTCAA A
Reverse sequence: TCTCA CACCA CTGTT CATAA GATTA G

The sequence of the primer for amplification of 15-PGDH is not limited to the above sequence, and any primer sequence complementary to a gene sequence of 15-PGDH can be adopted.

As an instrument used for performing quantitative PCR, for example, ABI Prism 7000 Sequence Detection System (Applied Biosystems) can be used.

mRNA of 15-PGDH may be quantified as a relative value with respect to an expression level of a housekeeping gene.

As the housekeeping gene, for example, GAPDH, β-actin, or β2-microglobulin HPRT1 can be used.

A quantitative value of mRNA of 15-PGDH is an index for success or failure of induction of labor.

From the viewpoint of improving the accuracy of prediction of success or failure of induction of labor, as an index for success or failure of induction of labor, a value obtained by dividing a quantitative value of mRNA of 15-PGDH by a quantitative value of mRNA of cyclooxygenase-2 (COX-2), which is a rate-limiting enzyme for prostaglandin production, (quantitative value of mRNA of 15-PGDH/quantitative value of mRNA of COX-2) may be used.

A testing reagent to predict success or failure of induction of labor (that is, a testing reagent for prediction of success or failure of induction of labor) containing a primer for 15-PGDH as described above is the testing reagent of the present invention.

The testing method according to the first embodiment of the present invention preferably further includes an evaluation step of evaluating a magnitude of the expression level of 15-PGDH in the specimen by comparing a signal of the expression level of 15-PGDH detected with an index of success or failure of induction of labor related to the signal.

In the testing method according to the first embodiment of the present invention, the signal of the expression level of 15-PGDH may be a relative value of the expression level of 15-PGDH based on an amount of total protein in a specimen.

The signal of the expression level of 15-PGDH may be a value obtained by dividing the expression level of 15-PGDH by an expression level of prostaglandin.

The signal of the expression level of 15-PGDH may be a value obtained by dividing a quantitative value of mRNA of 15-PGDH by a quantitative value of mRNA of cyclooxygenase-2 (COX-2), which is a rate-limiting enzyme for prostaglandin production, (quantitative value of mRNA of 15-PGDH/quantitative value of mRNA of COX-2).

The evaluation in the above step is, for example, to evaluate that induction of labor is unlikely to be successful in an individual from which the specimen is collected when a quantitative value of 15-PGDH is larger than a predetermined numerical value as an index of success or failure of induction of labor, and to evaluate that induction of labor is likely to be successful in an individual from which the specimen is collected when a quantitative value of 15-PGDH is smaller than a predetermined numerical value as an index of success or failure of induction of labor.

The index of success or failure of induction of labor can be arbitrarily set according to a method for measuring a signal of the expression level of 15-PGDH.

For example, the signal of the expression level of 15-PGDH may be a quantitative signal or a qualitative signal.

The signal of the expression level of 15-PGDH may be an expression level of 15-PGDH as a protein or an expression level of mRNA of 15-PGDH. The signal of the expression level of 15-PGDH may be a Ct value in quantitative PCR using a collected specimen and/or a value based on a Ct value in quantitative PCR using a collected specimen. The signal of the expression level of 15-PGDH may be color development intensity in an enzymatic reaction using a collected specimen.

In the present description, the "index of success or failure of induction of labor related to the signal" may be, for example, a cut-off value or a reference value (that is, an index derived from the "expression level of 15-PGDH") obtained based on data of a correlation between success or failure of induction of labor in a large number of subjects and the expression level of 15-PGDH.

(Second embodiment)

A testing method according to a second embodiment of the present invention is a testing method using an expression level of 15-PGDH as an index for preterm birth, the testing method including a step of detecting the expression level of 15-PGDH in a specimen, the specimen being a vaginal secretion.

In the testing method according to the second embodiment of the present invention, a vaginal secretion is used as a specimen.

Therefore, the testing method is non-invasive and less burdensome to a pregnant woman.

The testing method according to the second embodiment of the present invention includes a step of detecting an expression level of 15-PGDH in a vaginal secretion.

The expression level of 15-PGDH in a vaginal secretion can be an index for preterm birth.

Therefore, by using the testing method according to the second embodiment of the present invention, it is possible to predict whether or not preterm birth occurs with high reliability and simply.

A reason why the expression level of 15-PGDH is an index for preterm birth is considered to be due to the following action.

15-PGDH functions as a prostaglandin-inactivating enzyme. Therefore, since prostaglandin has a function of promoting delivery, 15-PGDH acts suppressively on delivery.

Therefore, when the expression level of 15-PGDH is low in a vagina, preterm birth is predicted to be likely to occur.

In the testing method according to the second embodiment of the present invention, when preterm birth is determined to be likely to occur, it is possible to reduce a risk of preterm birth by rapidly performing hospitalization management and preterm labor treatment by administering, for example, ritodrine hydrochloride, magnesium sulfate, and nifedipine, which are uterine contraction inhibitors.

In the testing method according to the second embodiment of the present invention, the vaginal secretion is preferably a secretion from an epithelium of a cervix and/or a vaginal squamous epithelium.

By using these vaginal secretions, it is possible to predict susceptibility to preterm birth with higher reliability.

In the testing method according to the second embodiment of the present invention, the vaginal secretion is preferably a secretion derived from a primiparous woman.

When the vaginal secretion is a secretion derived from a primiparous woman, it is possible to predict susceptibility to preterm birth with higher reliability.

In the testing method according to the second embodiment of the present invention, the detection of 15-PGDH may be detection by immunological measurement or detection by gene-related testing.

In the testing method according to the second embodiment of the present invention, when the detection of 15-PGDH is detection by immunological measurement, a step of quantifying the 15-PGDH is preferably included.

In the testing method according to the second embodiment of the present invention, when the detection of 15-PGDH is detection by gene-related testing, a step of quantifying mRNA of the 15-PGDH is preferably included.

In the testing method according to the second embodiment of the present invention, the detection of 15-PGDH by immunological measurement and the detection of 15-PGDH by gene-related testing can be performed by the same method as the detection of 15-PGDH by immunological measurement and the detection of 15-PGDH by gene-related testing in the testing method according to the first embodiment of the present invention.

A testing reagent for prediction of preterm birth used for such immunological measurement and gene-related testing, the testing reagent including an anti-15-PGDH antibody or a primer for 15-PGDH is the testing reagent of the present invention.

The testing method according to the second embodiment of the present invention preferably further includes an evaluation step of evaluating a magnitude of the expression level of 15-PGDH in the specimen by comparing a signal of the expression level of 15-PGDH detected with an index of preterm birth related to the signal.

The evaluation in the above step is, for example, to evaluate that preterm birth is unlikely to occur in an individual from which the specimen is collected when a quantitative value of 15-PGDH is larger than a predetermined numerical value as an index of preterm birth, and to evaluate that preterm birth is likely to occur in an individual from which the specimen is collected when a quantitative value of 15-PGDH is smaller than a predetermined numerical value as an index of preterm birth.

The index of preterm birth can be arbitrarily set according to a method for measuring a signal of the expression level of 15-PGDH.

For example, the signal of the expression level of 15-PGDH may be a quantitative signal or a qualitative signal.

The signal of the expression level of 15-PGDH may be an expression level of 15-PGDH as a protein or an expression level of mRNA of 15-PGDH. The signal of the expression level of 15-PGDH may be a Ct value in quantitative PCR using a collected specimen and/or a value based on a Ct value in quantitative PCR using a collected specimen. The signal of the expression level of 15-PGDH may be color development intensity in an enzymatic reaction using a collected specimen.

In the present description, the "index of preterm birth related to the signal" may be, for example, a cut-off value or a reference value (that is, an index derived from the "expression level of 15-PGDH") obtained based on data of a correlation between whether or not preterm birth occurs in a large number of subjects and the expression level of 15-PGDH.

As in the first embodiment, as the signal of the expression level of 15-PGDH, a relative value of 15-PGDH based on an amount of total protein in a specimen may be used.

As the signal of the expression level of 15-PGDH, a value obtained by dividing a quantitative value of 15-PGDH in a specimen by a quantitative value of prostaglandin in the specimen (quantitative value of 15-PGDH/quantitative value of prostaglandin) may be used.

As the signal of the expression level of 15-PGDH, a value obtained by dividing a quantitative value of mRNA of 15-PGDH by a quantitative value of mRNA of cyclooxygenase-2 (COX-2), which is a rate-limiting enzyme for prostaglandin production, (quantitative value of mRNA of 15-PGDH/quantitative value of mRNA of COX-2) may be used.

(Third embodiment)

A testing method according to a third embodiment of the present invention is a testing method using an expression level of 15-PGDH as an index for preterm labor, the testing method including a step of detecting the expression level of 15-PGDH in a specimen, the specimen being a vaginal secretion.

In the testing method according to the third embodiment of the present invention, a vaginal secretion is used as a specimen. Therefore, the testing method is non-invasive and less burdensome to a pregnant woman.

The testing method according to the third embodiment of the present invention includes a step of detecting an expression level of 15-PGDH in a vaginal secretion.

The expression level of 15-PGDH in a vaginal secretion can be an index for preterm labor.

Therefore, by using the testing method according to the third embodiment of the present invention, it is possible to predict whether or not preterm labor occurs with high reliability and simply.

A reason why the expression level of 15-PGDH is an index for preterm birth is considered to be due to the following action.

Preterm labor is a state in which there is a regular uterine contraction and a risk of preterm birth is high.

Here, 15-PGDH functions as a prostaglandin-inactivating enzyme. Therefore, since prostaglandin has a function of promoting uterine contraction, 15-PGDH acts suppressively on uterine contraction.

Therefore, when the expression level of 15-PGDH is low in a vagina, preterm labor is predicted to be likely to occur.

In the testing method according to the third embodiment of the present invention, when preterm birth is determined to be likely to occur, it is possible to avoid putting a mother and a fetus at risk of life by performing, for example, stable hospitalization, administration of a uterine contraction inhibitor, and check of intrauterine infection (e.g., blood collection, maternal fever).

In the testing method according to the third embodiment of the present invention, the vaginal secretion is preferably a secretion from an epithelium of a cervix and/or a vaginal squamous epithelium.

By using these vaginal secretions, it is possible to predict susceptibility to preterm labor with higher reliability.

In the testing method according to the third embodiment of the present invention, the vaginal secretion is preferably a secretion derived from a primiparous woman.

When the vaginal secretion is a secretion derived from a primiparous woman, it is possible to predict susceptibility to preterm labor with higher reliability.

In the testing method according to the third embodiment of the present invention, the detection of 15-PGDH may be detection by immunological measurement or detection by gene-related testing.

In the testing method according to the third embodiment of the present invention, when the detection of 15-PGDH is detection by immunological measurement, a step of quantifying the 15-PGDH is preferably included.

In the testing method according to the third embodiment of the present invention, when the detection of 15-PGDH is detection by gene-related testing, a step of quantifying mRNA of the 15-PGDH is preferably included.

In the testing method according to the third embodiment of the present invention, the detection of 15-PGDH by immunological measurement and the detection of 15-PGDH by gene-related testing can be performed by the same method as the detection of 15-PGDH by immunological measurement and the detection of 15-PGDH by gene-related testing in the testing method according to the first embodiment of the present invention.

A testing reagent for prediction of preterm labor used for such immunological measurement and gene-related testing, the testing reagent including an anti-15-PGDH antibody or a primer for 15-PGDH is the testing reagent of the present invention.

The testing method according to the third embodiment of the present invention preferably further includes an evaluation step of evaluating a magnitude of the expression level of 15-PGDH in the specimen by comparing a signal of the expression level of 15-PGDH detected with an index of preterm labor related to the signal.

The evaluation in the above step is, for example, to evaluate that preterm labor is unlikely to occur in an individual from which the specimen is collected when a quantitative value of 15-PGDH is larger than a predetermined numerical value as an index of preterm labor, and to evaluate that preterm labor is likely to occur in an individual from which the specimen is collected when a quantitative value of 15-PGDH is smaller than a predetermined numerical value as an index of preterm labor.

The index of preterm labor can be arbitrarily set according to a method for measuring a signal of the expression level of 15-PGDH.

For example, the signal of the expression level of 15-PGDH may be a quantitative signal or a qualitative signal.

The signal of the expression level of 15-PGDH may be an expression level of 15-PGDH as a protein or an expression level of mRNA of 15-PGDH. The signal of the expression level of 15-PGDH may be a Ct value in quantitative PCR using a collected specimen and/or a value based on a Ct value in quantitative PCR using a collected specimen. The signal of the expression level of 15-PGDH may be color development intensity in an enzymatic reaction using a collected specimen.

In the present description, the "index of preterm labor related to the signal" may be, for example, a cut-off value or a reference value (that is, an index derived from the "expression level of 15-PGDH") obtained based on data of a correlation between whether or not preterm labor occurs in a large number of subjects and the expression level of 15-PGDH.

As in the first embodiment, as the signal of the expression level of 15-PGDH, a relative value of a proportion of 15-PGDH based on an amount of total protein in a specimen may be used.

As the signal of the expression level of 15-PGDH, a value obtained by dividing a quantitative value of 15-PGDH in a specimen by a quantitative value of prostaglandin in the specimen (quantitative value of 15-PGDH/quantitative value of prostaglandin) may be used.

As the signal of the expression level of 15-PGDH, a value obtained by dividing a quantitative value of mRNA of 15-PGDH by a quantitative value of mRNA of cyclooxygenase-2 (COX-2), which is a rate-limiting enzyme for prostaglandin production, (quantitative value of mRNA of 15-PGDH/quantitative value of mRNA of COX-2) may be used.

(Other embodiments)

In the testing method of the present invention, a method for measuring the expression level of 15-PGDH is not limited. For example, any of the methods for measuring the expression level of 15-PGDH described in (Method for measuring concentration of 15-PGDH using solid-phase carrier reagent (A1) and labeling reagent (B1)), <Quantification of mRNA of 15-PGDH by quantitative PCR in women in the third trimester>, <Quantification of 15-PGDH using anti-15-PGDH antibody in women in the third trimester (1)>, <Quantification of mRNA of 15-PGDH in subjects who experienced preterm birth>, and <Quantification of 15-PGDH in subjects who experienced preterm birth> described in Examples described later is included in the method for measuring the expression level of 15-PGDH in the testing method of the present invention.

When a correlation coefficient of the measured expression level of 15-PGDH is R = 0.3 to 1.0 in comparison between these methods for measuring the expression level of 15-PGDH and other methods for measuring the expression level of 15-PGDH, the other methods for measuring the expression level of 15-PGDH are included in the method for measuring the expression level of 15-PGDH in the testing method of the present invention. The correlation coefficient R is preferably 0.4 to 1.0, more preferably 0.45 to 1.0.

EXAMPLES

Hereinafter, the testing method of the present invention is further described with reference to Examples, but the testing method of the present invention is not limited thereto.

<15-PGDH immunohistostaining>

In order to confirm whether or not 15-PGDH is expressed in utero during pregnancy, and in which tissue the 15-PGDH is expressed if the 15-PGDH is expressed, the following operations were performed.

(1) From two cases of pregnant patients with cervical cancer, uterine tissue was obtained by uterine extraction simultaneously with cesarean section at the time of delivery.
(2) From the obtained uterine tissue, a cervix of a normal site different from the site of cervical cancer was cut out, and the cervical tissue piece was subjected to paraffin fixation.
(3) The paraffin-fixed cervical tissue piece was sliced into a thickness of 5 um to obtain a tissue section. The tissue section was then deparaffinized in xylene.
(4) The tissue section was placed in a 10 mM citrate buffer (pH 6.0) and boiled in a microwave oven for 20 minutes. Thereafter, the tissue section was allowed to stand for 30 to 40 minutes until the temperature reached room temperature.
(5) The tissue section was washed 3 times with PBS (phosphate buffered saline obtained by dissolving 160 g of NaCl, 4 g of KCl, 28.8 g of $Na_2HPO_4$, and 4.8 g of $KH_2PO_4$ in 2 L of distilled water, adjusting the pH to 7.4, and sterilizing the solution by autoclaving).
(6) The tissue section was immersed in 3% (v/v) hydrogen peroxide water for 30 minutes at room temperature.
(7) The tissue section was washed 3 times with PBS.
(8) The tissue section was blocked for 15 minutes at room temperature using HISTOFINE Endogenous Avidin/Biotin Blocking Kit (#415041).
(9) The tissue section was washed 3 times with PBS.
(10) The tissue section was blocked with 10 wt% normal goat serum (using goat serum manufactured by Thermo Fisher Scientific, Inc.) for 30 minutes at room temperature.
(11) A primary antibody (15-PGDH Rabbit IgG (NB200-179, 2 mg/mL) diluted 200 times with a diluent (solution diluted with PBS so that BSA and Triton were 1% (v/v) and 0.3% (v/v), respectively)) was added to 50 μL of a blocking solution (10 wt% normal goat serum), and the mixture was allowed to stand at 4°C for 16 hours.
(12) The tissue section was taken out from the blocking solution and washed 3 times with 50 mL of PBS, then 50 μL of a secondary antibody (HISTOFINE Simple Stain MAX-PO(R), #424141) was added to the tissue section, and the tissue section was allowed to stand at room temperature for 20 minutes.
(13) The tissue section was removed from the reaction solution and washed 3 times with 50 mL of PBS.
(14) 50 μL of diaminobenzidine (DAB) was added to the tissue section and allowed to stand at room temperature for 3 to 4 minutes.
(15) The tissue section was washed 3 times with 50 mL of PBS.

(16) The tissue section was placed in a hematoxylin solution and allowed to stand for 5 minutes, then washed with running water, and the tissue section was embedded.

The embedded tissue section was observed with a light microscope. Light micrographs of the tissue section are shown in figures.

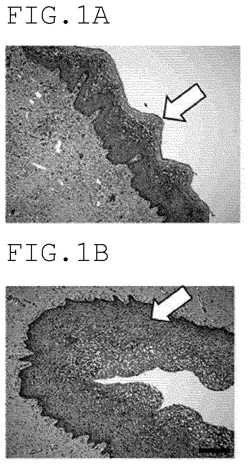

FIGS. 1A and 1B are photographs after immunohistostaining of a cervical tissue using an anti-15-PGDH antibody.

As shown in FIGS. 1A and 1B, 15-PGDH was observed to be expressed in squamous epithelial cells of a cervix (see arrows in FIGS. 1A and 1B).

An expression site of 15-PGDH was confirmed by the following method.

(Preparation of specimen: specimen collected from placenta)

(1) A placenta tissue was collected in a 2 cm square from a placenta of a cesarean section case (case A), and then washed with PBS.

(2) The placenta tissue was rapidly frozen with liquid nitrogen, and then the tissue was crushed using a Bessman tissue pulverizer. The crushed tissue was dissolved in 150 $\mu$L of a RIPA buffer (solution prepared by dissolving 8.766 g of NaCl, 10 mL of NP40, 5 g of sodium deoxycholate, 1 g of sodium dodecyl sulfate, and 6.055 g of trishydroxymethylaminomethane in distilled water to make a total amount of 1 L, and adjusting the pH to 7.4) containing a protease inhibitor of Complete mini (Roche, #11836153001, 1 tablet dissolved to 10 mL of RIPA buffer) to produce a protein solution.

(Preparation of specimen: specimen collected from amnion)

A specimen was obtained in the same manner except that the step of "(1)" was changed to the following step of "(1-1)" in the above (Preparation of specimen: specimen collected from placenta).

(1-1) From the placenta of a cesarean section case in the above (Preparation of specimen: specimen collected from placenta), fetal membranes were collected in a 5 cm square, washed once with PBS, and then an amnion was manually peeled off.

(Preparation of specimen: specimen collected from chorion)

A specimen was obtained in the same manner except that the step of "(1-1)" was changed to the following step of "(1-2)" in the above (Preparation of specimen: specimen collected from amnion).

(1-2) For the fetal membranes (choriodecidua) after collecting the amnion in the above (Preparation of specimen: specimen collected from amnion), a chorion was collected in a 5 cm square on a glass plate using a slide glass.

(Preparation of specimen: specimen collected from decidua)

A specimen was obtained in the same manner except that the step of "(1-2)" was changed to the following step of "(1-3)" in the above (Preparation of specimen: specimen collected from chorion).

(1-3) From the choriodecidua in the above (Preparation of specimen: specimen collected from chorion), the remaining tissue (decidua) after removal of the chorion was obtained at 5 cm square.

A specimen was collected in the same manner from a placenta, a chorion, and a decidua of another cesarean section case (case B) to produce a protein solution.

<Detection of 15-PGDH by Western blot>

Using a commercially available 15-PGDH antibody, it was confirmed whether 15-PGDH was expressed in a specimen (protein solution) at each site.

(1) SDS-PAGE

A total protein concentration in each protein solution was measured by BCA assay using Pierce BCA Protein Assay Kit (#23227, manufactured by Thermo Fisher Scientific, Inc.).

Based on the measured total protein concentration, a protein solution containing only 8 $\mu$g of protein was

collected and placed in a 1.5 mL Eppendorf tube. Thereafter, a RIPA buffer was further added to the Eppendorf tube to make a total solution amount of 12 μL.

Thereafter, 3 μL of a loading buffer [5 μL of 2-mercaptoethanol and a sample buffer (mixture of 3.125 mL of 1 M Tris-HCl buffer (pH 6.8), 5 mL of glycerol, and 1.25 mg of bromophenol blue)] was added to the Eppendorf tube to make a total solution amount of 15 μL.

Thereafter, the Eppendorf tube was heated at 95°C for 5 minutes to prepare a sample for SDS-PAGE.

Next, the sample for SDS-PAGE was applied to a 12% SDS-polyacrylamide gel, and electrophoresed at 150 V for 60 minutes.

(2) Western blot

The gel after electrophoresis was transferred to a PVDF membrane at 55 V for 1 hour.

The PVDF membrane was washed by performing one operation of immersing the PVDF membrane in 50 mL of Tris-TBST (solution prepared by dissolving 1.211 g of trishydroxymethylaminomethane, 8.766 g of NaCl, and 1 mL of Tween 20 in 1 L of distilled water and adjusting the pH to 7.4) and slowly shaking the PVDF membrane at room temperature for 3 minutes.

The PVDF membrane was immersed in 15 mL of 5% BSA/TBST (solution prepared by dissolving 5 g of bovine serum albumin (#01860-07, manufactured by Nacalai Tesque, Inc.) in 100 mL of the above Tris-TBST), and slowly shaken at room temperature for 1 hour to perform blocking treatment.

Next, a primary antibody [15-PGDH antibody (NB200-179, manufactured by Novus Biologicals) diluted 5000 times with 5% BSA/TBST] was added, and an antigen-antibody reaction was performed at 4°C for 16 hours.

Next, the PVDF membrane was washed by performing an operation of immersing the PVDF membrane in 50 mL of Tris-TBST and slowly shaking the PVDF membrane at room temperature for 3 minutes 3 times.

Next, a secondary antibody [HRP-Rabbit (#1706515, manufactured by Bio-Rad Laboratories, Inc.) diluted 10000 times with 5% BSA/TBST] was added, and an antigen-antibody reaction was performed at normal temperature for 1 hour.

Next, the PVDF membrane was washed by performing an operation of immersing the PVDF membrane in 50 mL of Tris-TBST and slowly shaking the PVDF membrane at room temperature for 3 minutes 3 times.

Thereafter, a chemiluminescent reaction was performed on the PVDF membrane using Pierce (trademark) ECL Plus Western Blotting Substrate (#32132, manufactured by Thermo Fisher Scientific, Inc.) for 1 minute.

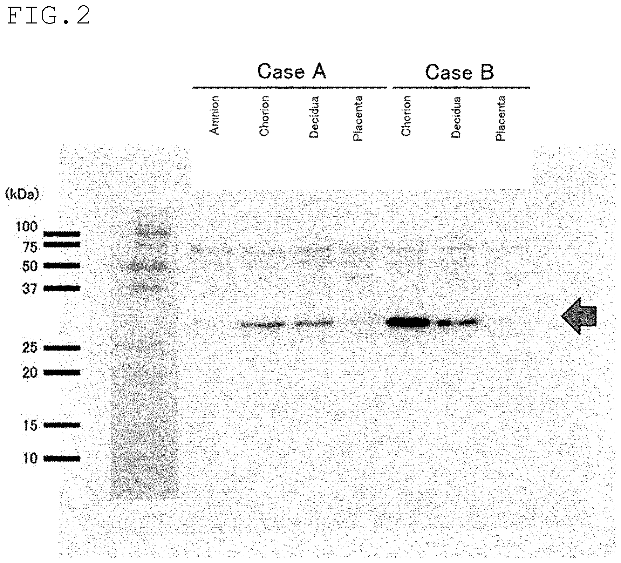

The PVDF membrane was photographed. The results are shown in FIG. 2.

FIG. 2 is a photograph of a PVDF membrane when Western blot is performed in a test for confirming an expression site of 15-PGDH. In FIG. 2, the band at the arrow indicates 15-PGDH.

FIG. 2 demonstrates that almost no 15-PGDH can be detected from an amnion, and 5-PGDH can be detected from a placenta, a decidua, and a chorion, and the expression level increases in this order.

<Confirmation of behavior of expression level of 15-PGDH from pregnancy to delivery>

In order to confirm a behavior of change in the expression level of 15-PGDH between pregnancy and delivery, the following operation was performed.

(Method for collecting specimen)

7 pregnant women were taken as subjects (Cases 1 to 7), and a vaginal secretion (including peeled epithelial tissue of cervix and vaginal squamous epithelial tissue) was collected using a sterilized cotton swab at the first, second, and third trimester of pregnancy using a speculum.

Next, the cotton swab to which the vaginal secretion was attached was put into a cryotube containing 1.5 mL of PBS and stirred well to extract the vaginal secretion. Thereafter, the content of the cryotube was equally divided into three cryotubes (0.5 mL per cryotube), and stored at - 80°C until use for testing.

(Measurement of total protein concentration)

Using one of the three cryotubes, a total protein concentration in the specimen was measured by BCA assay using Pierce BCA Protein Assay Kit (#23227, manufactured by Thermo Fisher Scientific, Inc.). The obtained total protein concentration was divided by 1 mg/mL, and the reciprocal thereof was used as a correction coefficient.

(Measurement of concentration of PGE2)

Using one of the three cryotubes, a concentration of PGE2 contained in the specimen was measured using DetectX Prostaglandin E2 Multi-Format ELISA Kit (Arbor Assays Inc.).

(Quantification of concentration of 15-PGDH)

Using one of the three cryotubes, centrifugation was performed under conditions of 12000 × G, 10 minutes, and 4°C to obtain a pellet.

The obtained pellet was dissolved in 250 µL of a RIPA buffer containing a protease inhibitor (Complete mini) [10 mL of a RIPA buffer (solution prepared by dissolving 8.766 g of NaCl, 10 mL of NP40, 5 g of sodium deoxycholate, 1 g of sodium dodecyl sulfate, and 6.055 g of trishydroxymethylaminomethane in distilled water to make a total amount of 1 L, and adjusting the pH to 7.4) and 1 tablet of Complete mini (Roche, #11836153001)] to produce a protein solution, and the solution was allowed to stand at 4°C for 10 minutes.

Using the protein solution as a specimen, a first anti-15-PGDH monoclonal antibody was used as a solid phase, and a second anti-15-PGDH antibody was used as a labeled antibody, and a concentration of 15-PGDH was measured by the (Method for measuring concentration of 15-PGDH using solid-phase carrier reagent (A1) and labeling reagent (B1)) described later.

Each measurement result is shown in Table 1.

In Table 1, the 15-PGDH correction value is a value obtained by multiplying a concentration of 15-PGDH of each sample by the correction coefficient. The same applies to the PGE2 correction value.

[Table 1]

| Case | Number of weeks of gestation | Number of weeks until delivery | Correction coefficient | 15-PGDH (pg/mL) | 15-PGDH correction value (pg/mL) | PGE2 (pg/mL) | PGE2 correction value (pg/mL) | 15-PGDH/PGE2 |
|---|---|---|---|---|---|---|---|---|
| 1 | 20 | 19 | 0.1607 | 1271.4 | 204.3 | 49.9 | 8.0 | 25.5 |
| | 28 | 11 | 0.3501 | 2224.8 | 778.8 | 135.6 | 47.5 | 16.4 |
| | 39 | 0 | 0.7158 | 248.9 | 178.2 | 45.9 | 32.9 | 5.4 |
| 2 | 21 | 19 | 0.6341 | 895.1 | 567.6 | 8.9 | 5.6 | 100.7 |
| | 31 | 9 | 0.2415 | 1229.3 | 296.9 | 160.3 | 38.7 | 7.7 |
| | 40 | 0 | 0.4241 | 589.7 | 250.1 | 222.4 | 94.3 | 2.7 |
| 3 | 21 | 19 | 0.1622 | 3034.2 | 492.1 | 46.0 | 7.5 | 66.0 |
| | 30 | 10 | 0.3934 | 1513.5 | 595.4 | 87.4 | 34.4 | 17.3 |
| | 40 | 0 | 1.4815 | 197.3 | 292.3 | 87.1 | 129.0 | 2.3 |
| 4 | 29 | 11 | 0.6863 | 699.7 | 480.2 | 197.7 | 135.7 | 3.5 |
| | 32 | 8 | 0.5760 | 1166.5 | 671.9 | 22.8 | 13.1 | 51.3 |
| | 40 | 0 | 0.5378 | 1272.9 | 684.6 | 1993.6 | 1072.2 | 0.6 |
| 5 | 29 | 8 | 0.8508 | 1403.7 | 1194.2 | 290.4 | 247.1 | 4.8 |
| | 37 | 0 | 0.3713 | 1448.6 | 537.8 | 164.8 | 61.2 | 8.8 |
| 6 | 20 | 19 | 0.8514 | 2171.2 | 1848.6 | 189.6 | 161.4 | 11.5 |
| | 30 | 9 | 0.2311 | 1478.4 | 341.6 | 121.1 | 28.0 | 12.2 |
| | 39 | 0 | 2.3810 | 243.5 | 579.8 | 500.0 | 1190.5 | 0.5 |
| 7 | 21 | 18 | 0.3511 | 1751.7 | 615.1 | 5.2 | 1.8 | 335.4 |
| | 28 | 9 | 0.7496 | 1713.2 | 1284.1 | 8.6 | 6.4 | 199.7 |
| | 39 | 0 | 0.9940 | 660.9 | 657.0 | 232.9 | 231.5 | 2.8 |

It was confirmed that the concentration of 15-PGDH decreased from about 10 weeks before delivery to the intrapartum period in 5 of 7 cases (Cases 1 to 3 and 6 to 7) .

It was confirmed that the 15-PGDH correction value decreased from about 10 weeks before delivery to the intrapartum period in 5 of 7 cases (Cases 1 to 3, 5, and 7) .

It was confirmed that the PGE2 correction value increased from about 10 weeks before delivery to the intrapartum period in 5 of 7 cases (Cases 2 to 4 and 6 to 7) .

It was confirmed that the value of 15-PGDH/PGE2 decreased from about 10 weeks before delivery to the intrapartum period in 6 of 7 cases (Cases 1 to 4 and 6 to 7) .

From a mechanism in which PGE2 is inactivated when an amount of 15-PGDH is large, and an amount of PGE2 increases when an amount of 15-PGDH is small, it is estimated that 15-PGDH/PGE2 has a small value in the intrapartum period.

From these results, it can be confirmed that the concentration of 15-PGDH, the concentration correction value of 15-PGDH, and the value of 15-PGDH/PGE2 tend to decrease from about 10 weeks before delivery. As described later, by performing the testing method of the present invention, it is possible to predict success or failure of induction of labor, preterm birth, and preterm labor on the basis of the concentration of 15-PGDH, the concentration correction value of 15-PGDH, and the value of 15-PGDH/PGE2 in women in the third trimester.

In view of these, in the testing method of the present invention, a woman at or after 10 weeks before the expected date of delivery is preferably subjected to testing from the viewpoint of increasing the accuracy.

(Method for measuring concentration of 15-PGDH using solid-phase carrier reagent (A1) and labeling reagent (B1))

In this measurement method, 15-PGDH is quantified using a first anti-15-PGDH monoclonal antibody as a solid phase and a second anti-15-PGDH antibody as a labeled antibody. A specific method is shown below.

Production of magnetic particles (E-1):

In a reaction vessel, 2.7 parts of iron(III) chloride hexahydrate, 1.0 part of iron(II) chloride tetrahydrate, and 375 parts of water were charged and dissolved, and the temperature was raised to 50°C. While maintaining the temperature at 50°C to 55°C under stirring, a solution obtained by mixing 3.8 parts of 25% aqueous ammonia and 100 parts of water was added dropwise over 1 hour, and the mixture was stirred for 1 hour after the dropwise addition to obtain magnetite particles (C-1) in water. To the obtained magnetite particles (C-1), 10.5 parts of oleic acid as a dispersant (K-1) was added, and stirring was continued for 2 hours. After cooling to room temperature, the magnetite particles to which oleic acid was adsorbed, which were obtained by solid-liquid separation by decantation, were washed 3 times with 50 parts of water. The obtained magnetite particles to which oleic acid was adsorbed were charged in a vessel, and 5.7 parts of decane (M-1) and 2.2 parts of tetraethoxysilane (N-1) were added and mixed to prepare a dispersion (A-1).

To a reaction vessel, 39.0 parts of a 25% ammonia (Q-1) aqueous solution, 55.4 parts of isopropanol (L-1), 2.9 parts of sorbitan monooleate ("IONET S-80" manufactured by Sanyo Chemical Industries, Ltd.), and 2.0 parts of polyoxyethylene (addition moles: 20 mol) alkyl ether ("EMULMIN 200" manufactured by Sanyo Chemical Industries, Ltd.) (P-1) were added and mixed using CLEARMIX (manufactured by M Technique Co., Ltd.). After the temperature was raised to 50°C, while stirring the mixture at a rotational speed of CLEARMIX of 6,000 rpm, the dispersion (A-1) was added dropwise over 1 hour, and then the mixture was reacted at 50°C for 1 hour. After the reaction, centrifugation was performed at 2,000 rpm for 5 minutes to remove a supernatant in which fine particles were present. 50 parts of water was added to the obtained solid phase to disperse the particles, and the mixture was centrifuged at 1,000 rpm for 10 minutes, and then an operation of removing a supernatant in which fine particles were present was performed 10 times. Subsequently, 50 parts of water was added to the obtained solid phase to disperse the particles, and the mixture was centrifuged at 500 rpm for 5 minutes to precipitate particles having a large particle size, and a supernatant (1) containing particles having a target particle size was recovered. 50 parts of water was added to the remaining solid phase, and the mixture was centrifuged at 500 rpm for 5 minutes, and then an operation of recovering a supernatant (2) was performed twice to recover particles having a desired particle size present in the solid phase. Next, for the supernatants (1) and (2), particles were magnetically collected using a magnet, and the magnetically collected particles were dried at 80°C for 8 hours to obtain magnetic particles (E-1).

Production of solid-phase carrier reagent (A1):

To a lidded polystyrene bottle containing 40 mL of a 1 wt% γ-aminopropyltriethoxysilane-containing aqueous solution, 40 mg of the magnetic particles (E-1) produced as described above was added, the mixture was reacted at 25°C for 1 hour, silica particles were magnetically collected with a neodymium magnet, and the liquid was removed by suction with an aspirator. Then, 40 mL of deionized water was added to cover the polystyrene bottle, and the polystyrene bottle was slowly inverted and stirred twice. Then, the silica particles were magnetically collected with a neodymium magnet, and the liquid was removed by suction with an aspirator to wash the silica particles. This washing operation was performed 5 times. Then, the washed silica particles were added to a lidded polystyrene bottle containing 40 mL of a 2 wt% glutaraldehyde-containing aqueous solution, and reacted at 25°C for 1 hour. Then, 40 mL of deionized water was added to cover the polystyrene bottle, and the polystyrene bottle was slowly inverted and stirred twice. Then, the silica particles were magnetically collected with a neodymium magnet, and the liquid was removed by suction with an aspirator to wash the silica particles. This washing operation was performed 10 times. Furthermore, the washed silica particles were added to a lidded polystyrene bottle containing 120 mL of a 0.02 M phosphate buffer (pH = 8.7) containing an anti-15-PGDH antibody (described as "first anti-15-PGDH antibody" for convenience) produced by a method described later at a concentration of 10 μg/mL, and reacted at 25°C for 1 hour. After the reaction, the silica particles were magnetically collected with a neodymium magnet, and then the first anti-15-PGDH antibody-containing phosphate buffer was removed. Next, 40 mg of the obtained silica particles was added to a lidded polyethylene bottle containing 40 mL of a 0.02 M phosphate buffer (pH 7.0) containing 1 wt% Block Ace [manufactured by DS Pharma Biomedical Co., Ltd.], and was immersed at 25°C for 12 hours. Thereby, a solid-phase carrier reagent (A1) containing silica particles on which the first anti-15-PGDH antibody was immobilized was obtained.

Production of labeling reagent (B1):

An anti-15-PGDH antibody (described as "second anti-15-PGDH antibody" for convenience) of another type than the first anti-15-PGDH antibody was selected.

Using the second anti-15-PGDH antibody and horseradish-derived POD [manufactured by TOYOBO CO., LTD.], a second anti-15-PGDH antibody labeled with POD was prepared by the method described in the literature (S. Yoshitake, M. Imagawa, E. Ishikawa, et al.; J. Biochem, Vol. 92, 1982, 1413-1424). This was diluted with a 0.02 M phosphate buffer containing a 1.0 wt% casein hydrolysate [manufactured by FUJIFILM Wako Pure Chemical Corporation] to a concentration of 0.5 μg/mL as a concentration of the second anti-15-PGDH antibody labeled with POD, and a labeling reagent (B1) was prepared and stored in a refrigerator (2°C to 10°C).

Production of buffer for immunoassay (H1):

A 0.02 M phosphate buffer (pH 7.0) containing 0.1 wt% bovine serum albumin (manufactured by Boval Company), 1 wt% EMULMIN L-90-S [manufactured by Sanyo Chemical Industries, Ltd.], 0.85 wt% sodium chloride [manufactured by FUJIFILM Wako Pure Chemical Corporation], and a 1.0 wt% casein hydrolysate [manufactured by FUJIFILM Wako Pure Chemical Corporation] was produced and stored in a refrigerator (2°C to 10°C).

Production of luminol luminescent reagent (E1):

0.7 g of a sodium salt of luminol [manufactured by Sigma-Aldrich Japan K. K.] and 0.1 g of 4-(cyanomethylthio)phenol were charged in a 1,000 mL volumetric flask. A 3-[4-(2-hydroxyethyl)-1-piperazinyl]propanesulfonic acid/sodium hydroxide buffer (10 mM, pH = 8.6) was charged so that the volume of the solution was 1,000 mL, and uniformly mixed at 25°C to produce a luminol luminescent reagent (E1). The luminol luminescent reagent was stored in a refrigerator (2°C to 10°C) until use for measurement.

Production of hydrogen peroxide solution (E2):

6.6 g of hydrogen peroxide [reagent special grade, concentration of 30 wt%, manufactured by Wako Pure Chemical Industries, Ltd.] was charged in a 1,000 mL volumetric flask. Deionized water was charged so that the volume of the solution was 1,000 mL, and uniformly mixed at 25°C to produce a hydrogen peroxide solution (E2). The hydrogen peroxide solution was stored in a refrigerator (2°C to 10°C) until use for measurement.

By performing the following steps (1) to (3) using the produced solid-phase carrier reagent (A1), labeling reagent (B1), buffer for immunoassay (H1), and hydrogen peroxide solution (E2), quantification of 15-PGDH contained in each specimen was measured.

Step (1)

Each 0.025 mL of the solid-phase carrier reagent (A1) was placed in a test tube, and the silica particles were collected with a neodymium magnet from the outside of the test tube for 10 seconds. The liquid in the test tube was removed with an aspirator, and the neodymium magnet was sufficiently separated from the side surface. 0.2 mL of the buffer for immunoassay (H) and 0.025 mL of various specimens as substances to be measured were placed in the test tube and mixed, and reacted at 37°C for 3 minutes in the test tube to form a complex (J-1) of an anti-15-PGDH antibody and 15-PGDH on anti-15-PGDH antibody-immobilized silica particles. After the reaction, the silica particles were collected with a neodymium magnet from the outside of the test tube for 10 seconds, the liquid in the test tube was removed with an aspirator, and the neodymium magnet was sufficiently separated from the side surface. Thereafter, 0.5 mL of physiological saline was added to disperse the silica particles, and the silica particles were magnetically collected. Then, a washing operation to remove the liquid with an aspirator was performed 3 times.

Step (2)

Subsequently, 0.1 mL of the labeling reagent (B1) of each immunoassay kit was injected into the test tube, and reacted at 37°C for 3 minutes in the test tube to form a labeled complex (L-1) of an anti-15-PGDH antibody, a 15-PGDH antibody, and a POD-labeled anti-15-PGDH antibody on anti-15-PGDH antibody-immobilized silica particles. After the reaction, the silica particles were collected with a neodymium magnet from the outside of the test tube for 10 seconds, the liquid in the test tube was removed with an aspirator, and the neodymium magnet was sufficiently separated from the side surface. Thereafter, 0.5 mL of physiological saline was added to disperse the silica particles, and the silica particles were magnetically collected. Then, a washing operation to remove the liquid with an aspirator was performed twice.

Step (3)

Next, 0.1 mL of the luminol luminescent reagent (E1) and 0.1 mL of the hydrogen peroxide solution (E2) were simultaneously added, and a luminescent reaction was caused at 37°C. An average amount of luminescence per 1 second in 40 to 45 seconds after the addition of the luminol luminescent reagent (E1) and the hydrogen peroxide solution (E2) was measured with a luminometer ["Lumat LB9507" manufactured by Berthold Japan K. K.].

In the same manner except that the following standard solutions were respectively used instead of "various specimens as substances to be measured" in the above "Step (1)", an average amount of luminescence was measured with a luminometer, and a calibration curve showing a relationship between an amount of luminescence and a concentration of 15-PGDH was created. From the obtained calibration curve, the measured concentration in the above "immunoassay" was determined. The results are shown in Table 1.

Standard solution: Human 15-PGDH/HPGD Recombinant Protein (manufactured by BiosPacific) prepared to have a concentration of 0 to 10,000 pg/mL (concentration: 0, 20, 100, 200, 500, 1,000, 3,000, 6,000, 10,000 pg/mL)

<Quantification of mRNA of 15-PGDH by quantitative PCR in women in the third trimester>

(1) Specimen collection

26 women in the third trimester were taken as subjects, and a vaginal secretion was collected using a sterilized cotton swab at the time of gynecological examination using a speculum, and a vaginal secretion (including peeled epithelial tissue of cervix and vaginal squamous epithelial tissue) was collected.

Next, the cotton swab to which the vaginal secretion was attached was put into a cryotube containing 1.5 mL of RNA later [RNA later Soln (#AM7021, manufactured by Invitrogen)] and stirred well to extract the vaginal secretion. Thereafter, the cryotube was stored at -80°C until use for testing.

(2) Induction of labor

After collection of the vaginal secretion, an oxytocin preparation was used for subjects to attempt induction of labor.

A dose of the oxytocin preparation was started at 1.5 miliunits/min. If no active labor occurs, the dose was increased by 1.5 miliunits/min every 30 minutes, and the oxytocin preparation was administered at a maximum dose of 20 miliunits/min. Success or failure of induction of labor was classified according to the following criteria.

Labor-induction success group: Active labor occurred within 48 hours from the start of use of the oxytocin preparation.

Labor-induction failure group: Active labor did not occur within 48 hours from the start of use of the oxytocin preparation.

Regarding active labor, "labor occurs regularly and continues until delivery of a fetus, and when the cycle is within 10 minutes or at a frequency of 6 times per hour", it is determined that "active labor occurred".

(3) Quantitative PCR

750 μL of the vaginal secretion dissolved in RNA later obtained by the operation of the above "(1) Specimen collection" was slowly thawed at 4°C, shaken so that the content was even, and then transferred to another 1.5 mL Eppendorf tube.

Next, centrifugation was performed under conditions of 12000 × G, 10 minutes, and 4°C to obtain a pellet.

Next, RNA was extracted from the obtained pellet using Sepasol RNA Super G (Nacalai Tesque, Inc., 09379-26).

Thereafter, a total RNA amount of the extracted RNA was measured using Thermo Scientific Nanodrop 1000.

Based on the measured total RNA amount, 1 ug of RNA was collected, and a cDNA library was produced using ReverTra Ace qPCR RT Master Mix (TOYOBO CO., LTD., FSQ-201) .

Quantitative PCR was performed using the produced cDNA library, and the expression level of mRNA of 15-PGDH was quantified as a threshold cycle (Ct) value.

The quantitative PCR was performed in duplicate, and an average value of the obtained Ct values was taken as the expression level of mRNA of 15-PGDH in the subject.

THUNDERBIRD SYBR qPCR Mix (TOYOBO CO., LTD., QPS-201) was used as a reagent for quantitative PCR, and ABI Prism 7000 Sequence Detection System (Applied Biosystems) was used as an instrument.

The sequence of the primer for amplification of 15-PGDH used is as follows.

Sequence of primer for amplification of 15-PGDH:

Forward sequence: GCCGGTTTATTGTGCTTCAAA
Reverse sequence: TCTCACACCACTGTTCATAAGATTAG

Quantitative PCR was performed using the produced cDNA library, and the expression level of mRNA of GAPDH as a housekeeping gene was quantified as a Ct value.

The quantitative PCR was performed twice, and an average value of the obtained Ct values was taken as the expression level of mRNA of GAPDH in the subject.

As the reagent for quantitative PCR and the instrument, the same reagent and instrument as those described above were used.

The sequence of the primer for amplification of GAPDH used is as follows.

Sequence of primer for amplification of GAPDH:

Forward sequence: GGAGTCAACGGATTTGGTCGTA
Reverse sequence: CAACAATATCCACTTTACCAGAGTTA

Relative fold expression (rel to cal) was calculated based on the expression levels of mRNA of 15-PGDH and mRNA of GAPDH.

The relative fold expression can be calculated by the following formulas (1) to (4). The results are shown in Table 2.

$$\text{dCt} = [\text{Ct value of 15-PGDH}] - [\text{Ct value of GAPDH}] \cdots (1)$$

$$0\text{Ave} = \text{average value of dCt values in labor-induction failure group} \qquad (2)$$

$$\text{ddCt} = \text{dCt} - 0\text{Ave} \cdots (3)$$

$$[\text{rel to cal}] = 2^{-\text{ddCt}} \cdots (4)$$

[Table 2]

| Subject | Success or failure of induction of labor | Expression level of mRNA (Ct) | | dCt | ddCt | rel to cal |
|---|---|---|---|---|---|---|
| | | 15-PGDH | GAPDH | | | |
| 1-1 | Success | 21.60 | 22.98 | -1.38 | -1.04 | 2.06 |
| 1-2 | Success | 22.39 | 22.12 | 0.27 | 0.60 | 0.66 |
| 1-3 | Success | 24.73 | 19.67 | 5.06 | 5.40 | 0.02 |
| 1-4 | Success | 22.57 | 21.88 | 0.68 | 1.02 | 0.49 |
| 1-5 | Success | 24.53 | 21.13 | 3.40 | 3.74 | 0.07 |
| 1-6 | Success | 23.29 | 19.42 | 3.86 | 4.20 | 0.05 |
| 1-7 | Success | 20.94 | 21.22 | -0.28 | 0.06 | 0.96 |
| 1-8 | Success | 19.87 | 22.04 | -2.17 | -1.83 | 3.54 |
| 1-9 | Success | 21.78 | 21.36 | 0.43 | 0.76 | 0.59 |
| 1-10 | Success | 23.79 | 22.54 | 1.25 | 1.59 | 0.33 |
| 1-11 | Success | 21.55 | 22.10 | -0.55 | -0.21 | 1.16 |
| 1-12 | Success | 25.95 | 22.78 | 3.17 | 3.51 | 0.09 |
| 1-13 | Success | 20.21 | 20.62 | -0.41 | -0.07 | 1.05 |
| 1-14 | Success | 21.38 | 20.65 | 0.73 | 1.06 | 0.48 |
| 1-15 | Success | 22.15 | 22.21 | -0.06 | 0.28 | 0.82 |
| 1-16 | Success | 25.21 | 23.01 | 2.20 | 2.53 | 0.17 |
| 1-17 | Failure | 22.03 | 21.77 | 0.26 | 0.60 | 0.66 |
| 1-18 | Failure | 20.13 | 22.20 | -2.07 | -1.73 | 3.32 |
| 1-19 | Failure | 19.17 | 20.67 | -1.50 | -1.16 | 2.23 |
| 1-20 | Failure | 20.23 | 22.50 | -2.27 | -1.93 | 3.80 |
| 1-21 | Failure | 20.59 | 22.57 | -1.98 | -1.64 | 3.11 |
| 1-22 | Failure | 22.05 | 23.20 | -1.15 | -0.81 | 1.75 |
| 1-23 | Failure | 21.50 | 19.44 | 2.06 | 2.40 | 0.19 |
| 1-24 | Failure | 25.11 | 23.24 | 1.87 | 2.20 | 0.22 |
| 1-25 | Failure | 20.01 | 21.55 | -1.54 | -1.20 | 2.29 |
| 1-26 | Failure | 26.00 | 23.09 | 2.91 | 3.24 | 0.11 |
| "0Ave" was -0.34. | | | | | | |

Regarding the relative fold expression, respective subjects were divided into a labor-induction success group (16 points) and a labor-induction failure group (10 points), and a P value when a null hypothesis "there is no difference in quantitative values of mRNA of 15-PGDH between the labor-induction success group and the labor-induction failure group" was set was calculated, and the P value was 0.039.

That is, the value of relative fold expression was shown to be significantly lower in the labor-induction success group than that in the labor-induction failure group.

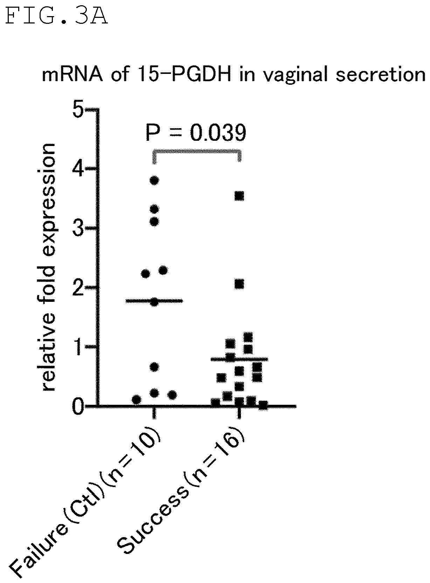

A distribution of values of relative fold expression in the labor-induction success group and the labor-induction failure group is shown in FIG. 3A.

FIG. 3A is a diagram showing a distribution of values of relative fold expression using quantitative values of mRNA of 15-PGDH in the labor-induction success group and the labor-induction failure group.

With "being in a state in which induction of labor is successful" as a definition of 15-PGDH positivity, sensitivity, specificity, a matching rate with clinical practice (accuracy), a positive predictive value, and a negative predictive value when a cut-off value was set to dCt value = -2 to 5 were calculated. The results are shown in Table 3.

[Table 3]

| Cut-off value (dCt value) | Sensitivity | Specificity | Matching rate with clinical practice | Positive predictive value | Negative predictive value |
|---|---|---|---|---|---|
| -2 | 94% | 20% | 65% | 65% | 67% |
| -1.5 | 94% | 50% | 77% | 75% | 83% |
| -1.3 | 88% | 50% | 73% | 74% | 71% |
| -1.2 | 88% | 50% | 73% | 74% | 71% |
| -1 | 88% | 60% | 77% | 78% | 75% |
| -0.9 | 88% | 60% | 77% | 78% | 75% |
| -0.7 | 88% | 60% | 77% | 78% | 75% |
| -0.5 | 81% | 60% | 73% | 76% | 67% |
| 0 | 63% | 60% | 62% | 71% | 50% |
| 0.5 | 50% | 70% | 58% | 73% | 47% |
| 1 | 38% | 70% | 50% | 67% | 41% |
| 1.5 | 31% | 70% | 46% | 63% | 39% |
| 2 | 31% | 80% | 50% | 71% | 42% |
| 2.5 | 25% | 90% | 50% | 80% | 43% |
| 3 | 25% | 100% | 54% | 100% | 45% |
| 4 | 6% | 100% | 42% | 100% | 40% |
| 5 | 6% | 100% | 42% | 100% | 40% |

When 15-PGDH positivity is determined under the above conditions, the cut-off value is preferably set to any value in a range of dCt value = -2.5 to 6, more preferably set to any value in a range of -2 to 5, still more preferably set to any value in a range of -1.5 to 3, particularly preferably set to any value in a range of more than -1.15 and -0.55 or less, particularly preferably set to any value in a range of -1.10 to -1.00 or less.

When the cut-off value is set using a dCt value, it can be determined that induction of labor is likely to be successful when the dCt value exceeds the cut-off value. That is, the dCt value can be used as an index of success or failure of induction of labor.

When the cut-off value is set to any value in a range of dCt value = -1.5 to -0.5, the sensitivity is 81 to 94%, the specificity is 50 to 60%, the matching rate with clinical practice (accuracy) is 73 to 77%, the positive predictive value is 74 to 78%, and the negative predictive value is 67 to 83%.

When the cut-off value is set to any value in a range of dCt value = 2 to 3, the sensitivity is 25 to 31%, the specificity is 80 to 100%, the matching rate with clinical practice (accuracy) is 50 to 54%, the positive predictive value is 71 to 100%, and the negative predictive value is 42 to 45%.

From the viewpoint of sensitivity, the cut-off value is preferably set to any value in a range of dCt value = - 2.5 to 0.5, more preferably set to any value in a range of -2 to -0.5.

From the viewpoint of specificity, the cut-off value is preferably set to any value in a range of dCt value = - 1.5 to 6, more preferably set to any value in a range of 0.5 to 6, still more preferably set to any value in a range of 0.5 to 5.

From the viewpoint of matching rate with clinical practice (accuracy), the cut-off value is preferably set to any value in a range of dCt value = -2.5 to 3, more preferably set to any value in a range of -2.5 to -0.5, still more preferably set to any value in a range of -2 to -0.5.

From the viewpoint of positive predictive value, the cut-off value is preferably set to any value in a range of dCt value = -2.5 to 5, more preferably set to any value in a range of -2.5 to 1 or any value in a range of 2 to 5, still more preferably set to any value in a range of -2 to 1 or any value in a range of 2 to 5.

From the viewpoint of negative predictive value, the cut-off value is preferably set to any value in a range of dCt value = -2 to 0, more preferably set to any value in a range of -2 to -0.5.

When the cut-off value is set to any value of dCt value = -0.5 or less, it is possible to prevent oversight of success of induction of labor with high probability.

When the cut-off value is set to any value of dCt value = 2 or more, it is possible to prevent oversight of failure of induction of labor with high probability.

Since a preferable cut-off value varies depending on the type of primer used for measurement and measurement conditions, the cut-off value is preferably appropriately set.

In <Quantification of 15-PGDH using anti-15-PGDH antibody in women in the third trimester (2)> described later, an amount of the specimen of subject 1-7 was small, and 15-PGDH could not be quantified.

Therefore, in order to compare an index of success or failure of induction of labor based on <Quantification of mRNA of 15-PGDH by quantitative PCR in women in the third trimester> with an index of success or failure of induction of labor based on <Quantification of 15-PGDH using anti-15-PGDH antibody in women in the third trimester (2)>, the subjects were divided into a labor-induction success group (15 points) excluding subject 1-7 and a labor-induction failure group (10 points). Then, a P value when a null hypothesis "there is no difference in quantitative values of mRNA of 15-PGDH between the labor-induction success group and the labor-induction failure group" was set was calculated, and the P value was 0.044.

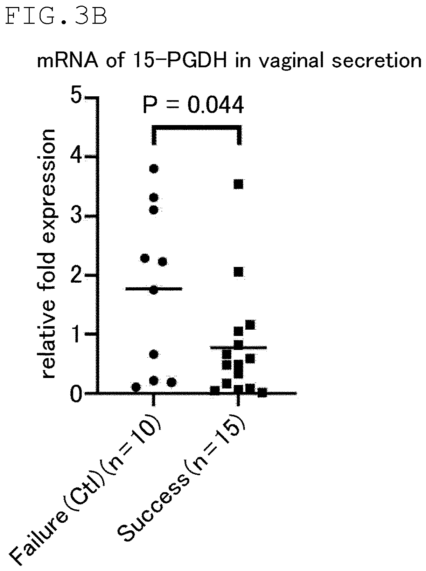

A distribution of values of relative fold expression in the labor-induction success group and the labor-induction failure group in this case is shown in FIG. 3B.

FIG. 3B is a diagram showing a distribution of values of relative fold expression using quantitative values of mRNA of 15-PGDH in the labor-induction success group (excluding subject 1-7) and the labor-induction failure group.

With "being in a state in which induction of labor is successful" as a definition of 15-PGDH positivity, sensitivity, specificity, a matching rate with clinical practice (accuracy), a positive predictive value, and a negative predictive value when a cut-off value was set to dCt value = -2 to 5 were calculated. The results are shown in Table 4.

[Table 4]

| Cut-off value (dCt value) | Sensitivity | Specificity | Matching rate with clinical practice | Positive predictive value | Negative predictive value |
|---|---|---|---|---|---|
| -2 | 93% | 20% | 64% | 64% | 67% |
| -1.5 | 93% | 50% | 76% | 74% | 83% |
| -1.3 | 87% | 50% | 72% | 72% | 71% |
| -1.2 | 87% | 50% | 72% | 72% | 71% |
| -1 | 87% | 60% | 76% | 76% | 75% |
| -0.9 | 87% | 60% | 76% | 76% | 75% |
| -0.7 | 87% | 60% | 76% | 76% | 75% |
| -0.5 | 80% | 60% | 72% | 75% | 67% |
| 0 | 67% | 60% | 64% | 71% | 55% |
| 0.5 | 53% | 70% | 60% | 73% | 50% |
| 1 | 40% | 70% | 52% | 67% | 44% |
| 1.5 | 33% | 70% | 48% | 63% | 41% |
| 2 | 33% | 80% | 52% | 71% | 44% |
| 2.5 | 27% | 90% | 52% | 80% | 45% |
| 3 | 27% | 100% | 56% | 100% | 48% |
| 4 | 7% | 100% | 44% | 100% | 42% |
| 5 | 7% | 100% | 44% | 100% | 42% |

Among the obtained data, using data of only primiparous women [subjects 1-3, 1-4, 1-6, 1-7, 1-10, 1-12, 1-13, 1-14, 1-15, 1-18, 1-20, 1-21, 1-22, 1-24, and 1-25 in Table 2 (labor-induction success group (9 points) and labor-induction failure group (6 points))], a P value was calculated by the same method as described above, and the P value was 0.00096.

That is, the value of relative fold expression was shown to be further significantly lower in the labor-induction success group than that in the labor-induction failure group in subjects of only primiparous women.

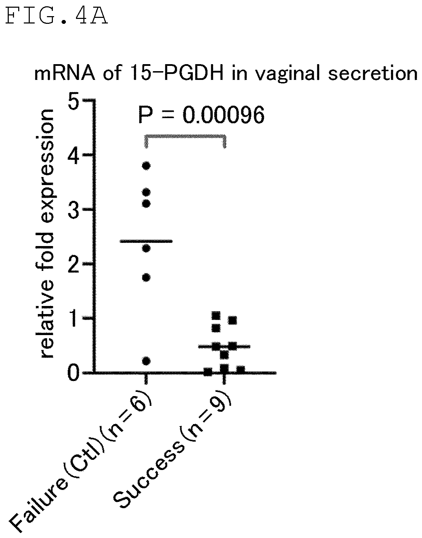

A distribution of values of relative fold expression in the labor-induction success group and the labor-induction failure group in subjects of only primiparous women is shown in FIG. 4A.

FIG. 4A is a diagram showing a distribution of values of relative fold expression using quantitative values of mRNA of 15-PGDH in the labor-induction success group and the labor-induction failure group in subjects of only primiparous women.

With "being in a state in which induction of labor is successful" as a definition of 15-PGDH positivity, sensitivity, specificity, a matching rate with clinical practice (accuracy), a positive predictive value, and a negative predictive value when a cut-off value was set to dCt value = -2 to 5 were calculated. The results are shown in Table 5.

[Table 5]

| Cut-off value (dCt value) | Sensitivity | Specificity | Matching rate with clinical practice | Positive predictive value | Negative predictive value |
|---|---|---|---|---|---|
| -2 | 100% | 33% | 73% | 69% | 100% |
| -1.8 | 100% | 50% | 80% | 75% | 100% |
| -1.5 | 100% | 67% | 87% | 82% | 100% |
| -1.3 | 100% | 67% | 87% | 82% | 100% |
| -1.2 | 100% | 67% | 87% | 82% | 100% |
| -1 | 100% | 83% | 93% | 90% | 100% |
| -0.9 | 100% | 83% | 93% | 90% | 100% |
| -0.7 | 100% | 83% | 93% | 90% | 100% |
| -0.5 | 100% | 83% | 93% | 90% | 100% |
| -0.3 | 89% | 83% | 87% | 89% | 83% |
| -0.1 | 78% | 83% | 80% | 88% | 71% |
| 0 | 67% | 83% | 73% | 86% | 63% |
| 0.5 | 67% | 83% | 73% | 86% | 63% |
| 0.7 | 56% | 83% | 67% | 83% | 56% |
| 1 | 44% | 83% | 60% | 80% | 50% |
| 1.5 | 33% | 83% | 53% | 75% | 45% |
| 2 | 33% | 100% | 60% | 100% | 50% |
| 3 | 33% | 100% | 60% | 100% | 50% |
| 4 | 11% | 100% | 47% | 100% | 43% |
| 5 | 11% | 100% | 47% | 100% | 43% |

When 15-PGDH positivity is determined under the above conditions, the cut-off value is preferably set to any value in a range of dCt value = -2.5 to 6, more preferably set to any value in a range of -2 to 5, still more preferably set to any value in a range of -2 to 3.

When the cut-off value is set to any value in a range of dCt value = -1.5 to -0.5, the sensitivity is 100%, the specificity is 67 to 83%, the matching rate with clinical practice (accuracy) is 87 to 93%, the positive predictive value is 82 to 90%, and the negative predictive value is 100%.

When the cut-off value is set to any value in a range of dCt value = 2 to 3, the sensitivity is 33%, the specificity is 100%, the matching rate with clinical practice (accuracy) is 60%, the positive predictive value is 100%, and the negative predictive value is 50%.

From the viewpoint of sensitivity, the cut-off value is preferably set to any value in a range of dCt value = - 2.5 to 0.7, more preferably set to any value in a range of -2.5 to -0.1, still more preferably set to any value in a range of -2 to -0.1.

From the viewpoint of specificity, the cut-off value is preferably set to any value in a range of dCt value = - 1.8 to 6, more preferably set to any value in a range of -1 to 6, still more preferably set to any value in a range of -1 to 5.

From the viewpoint of matching rate with clinical practice (accuracy), the cut-off value is preferably set to any value in a range of dCt value = -2.5 to 3, more preferably set to any value in a range of -2 to 0.7.

From the viewpoint of positive predictive value, the cut-off value is preferably set to any value in a range of dCt value = -2.5 to 5, more preferably set to any value in a range of -1.8 to 5.

From the viewpoint of negative predictive value, the cut-off value is preferably set to any value in a range of dCt value = -2 to 1, more preferably set to any value in a range of -2 to -0.1.

When the cut-off value is set to any value of dCt value = -0.5 or less, it is possible to prevent oversight of success of induction of labor with high probability.

When the cut-off value is set to any value of dCt value = -1 or more, it is possible to prevent oversight of failure of induction of labor with high probability.

Since a preferable cut-off value varies depending on the type of primer used for measurement and measurement conditions, the cut-off value is preferably appropriately set.

When Table 3 and Table 5 are compared, a maximum value of the matching rate with clinical practice is higher when the data of only primiparous women are used. Therefore, the above method is useful for predicting success or failure of induction of labor in primiparous women.

Also regarding the data of only primiparous women, only data of subject 1-7 were excluded, and a P value was calculated by the same method as described above. As a result, the P value was 0.0013.

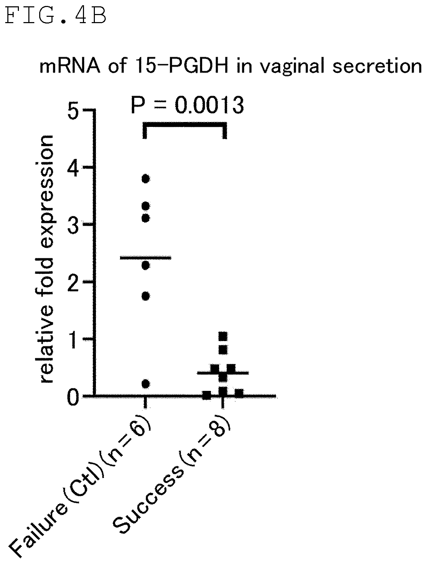

A distribution of values of relative fold expression in the labor-induction success group and the labor-induction failure group in this case is shown in FIG. 4B.

FIG. 4B is a diagram showing a distribution of values of relative fold expression using quantitative values of mRNA of 15-PGDH in the labor-induction success group (excluding subject 1-7) and the labor-induction failure group in subjects of only primiparous women.

With "being in a state in which induction of labor is successful" as a definition of 15-PGDH positivity, sensitivity, specificity, a matching rate with clinical practice (accuracy), a positive predictive value, and a negative predictive value when a cut-off value was set to dCt value = -2 to 5 were calculated. The results are shown in Table 6.

[Table 6]

| Cut-off value (dCt value) | Sensitivity | Specificity | Matching rate with clinical practice | Positive predictive value | Negative predictive value |
|---|---|---|---|---|---|
| -2 | 100% | 33% | 71% | 67% | 100% |
| -1.8 | 100% | 50% | 79% | 73% | 100% |
| -1.5 | 100% | 67% | 86% | 80% | 100% |
| -1.3 | 100% | 67% | 86% | 80% | 100% |
| -1.2 | 100% | 67% | 86% | 80% | 100% |
| -1 | 100% | 83% | 93% | 89% | 100% |
| -0.9 | 100% | 83% | 93% | 89% | 100% |
| -0.7 | 100% | 83% | 93% | 89% | 100% |
| -0.5 | 100% | 83% | 93% | 89% | 100% |
| -0.3 | 88% | 83% | 86% | 88% | 83% |
| -0.1 | 88% | 83% | 86% | 88% | 83% |
| 0 | 75% | 83% | 79% | 86% | 71% |
| 0.5 | 75% | 83% | 79% | 86% | 71% |
| 0.7 | 63% | 83% | 71% | 83% | 63% |
| 1 | 50% | 83% | 64% | 80% | 56% |
| 1.5 | 38% | 83% | 57% | 75% | 50% |
| 2 | 38% | 100% | 64% | 100% | 55% |
| 3 | 38% | 100% | 64% | 100% | 55% |
| 4 | 13% | 100% | 50% | 100% | 46% |
| 5 | 13% | 100% | 50% | 100% | 46% |

<Quantification of 15-PGDH using anti-15-PGDH antibody in women in the third trimester (1)>

(1) Specimen collection

18 women in the third trimester were taken as subjects, and a vaginal secretion was collected using a sterilized cotton swab at the time of gynecological examination using a speculum, and a vaginal secretion (including peeled epithelial tissue of cervix and vaginal squamous epithelial tissue) was collected.

Next, the cotton swab to which the vaginal secretion was attached was put into a cryotube containing 1.5 mL of PBS and stirred well to extract the vaginal secretion. Thereafter, the cryotube was stored at -80°C until use for testing.

(2) Induction of labor

After collection of the vaginal secretion, an oxytocin preparation was used for subjects to attempt induction of labor.

A dose of the oxytocin preparation was started at 1.5 miliunits/min. If no active labor occurs, the dose was increased by 1.5 miliunits/min every 30 minutes, and the oxytocin preparation was administered at a maximum dose of 20 miliunits/min. Success or failure of induction of labor was classified according to the following criteria.

Labor-induction success group: Active labor occurred within 48 hours from the start of use of the oxytocin preparation.

Labor-induction failure group: Active labor did not occur within 48 hours from the start of use of the oxytocin preparation.

(3) SDS-PAGE

The cryotube obtained in the above "(1) Specimen collection" was slowly thawed at 4°C, shaken so that the content was even, and then 750 $\mu$L of the content was transferred to a new cryotube.

Next, centrifugation was performed under conditions of 12000 × G, 10 minutes, and 4°C to obtain a pellet.

The obtained pellet was dissolved in 150 $\mu$L of a RIPA buffer containing a protease inhibitor (Complete mini) [10 mL of a RIPA buffer (solution prepared by dissolving 8.766 g of NaCl, 10 mL of NP40, 5 g of sodium deoxycholate, 1 g of sodium dodecyl sulfate, and 6.055 g of trishydroxymethylaminomethane in distilled water to make a total amount of 1 L, and adjusting the pH to 7.4) and 1 tablet of Complete mini (Roche, #11836153001)] to produce a protein solution.

A total protein concentration in the protein solution was measured by BCA assay using Pierce BCA Protein Assay Kit (#23227, manufactured by Thermo Fisher Scientific, Inc.).

Based on the measured total protein concentration, a protein solution containing only 8 $\mu$g of protein was collected and placed in a 1.5 mL Eppendorf tube. Thereafter, a RIPA buffer was further added to the Eppendorf tube to make a total solution amount of 12 $\mu$L.

Thereafter, 3 $\mu$L of a loading buffer [5 $\mu$L of 2-mercaptoethanol and a sample buffer (mixture of 3.125 mL of 1 M Tris-HCl buffer (pH 6.8), 5 mL of glycerol, and 1.25 mg of bromophenol blue)] was added to the Eppendorf tube to make a total solution amount of 15 $\mu$L.

Thereafter, the Eppendorf tube was heated at 95°C for 5 minutes to prepare a sample for SDS-PAGE.

Next, the sample for SDS-PAGE was applied to a 12% SDS-polyacrylamide gel, and electrophoresed at 150 V for 60 minutes.

(4) Western blot

The gel after electrophoresis was transferred to a PVDF membrane at 55 V for 1 hour.

The PVDF membrane was washed by performing one operation of immersing the PVDF membrane in 50 mL of Tris-TBST (solution prepared by dissolving 1.211 g of trishydroxymethylaminomethane, 8.766 g of NaCl, and 1 mL of Tween 20 in 1 L of distilled water and adjusting the pH to 7.4) and slowly shaking the PVDF membrane at room temperature for 3 minutes.

The PVDF membrane was immersed in 15 mL of 5% BSA/TBST (solution prepared by dissolving 5 g of bovine serum albumin (#01860-07, manufactured by Nacalai Tesque, Inc.) in 100 mL of the above Tris-TEST), and slowly shaken at room temperature for 1 hour to perform blocking treatment.

Next, a primary antibody [15-PGDH antibody (NB200-179, manufactured by Novus Biologicals) diluted 5000 times with 5% BSA/TBST] was added, and an antigen-antibody reaction was performed at 4°C for 16 hours.

Next, the PVDF membrane was washed by performing an operation of immersing the PVDF membrane in 50 mL of Tris-TBST and slowly shaking the PVDF membrane at room temperature for 3 minutes 3 times.

Next, a secondary antibody [HRP-Rabbit (#1706515, manufactured by Bio-Rad Laboratories, Inc.) diluted 10000 times with 5% BSA/TBST] was added, and an antigen-antibody reaction was performed at normal temperature for 1 hour.

Next, the PVDF membrane was washed by performing an operation of immersing the PVDF membrane in 50 mL of Tris-TBST and slowly shaking the PVDF membrane at room temperature for 3 minutes 3 times.

Thereafter, a chemiluminescent reaction was performed on the PVDF membrane using Pierce (trademark) ECL Plus Western Blotting Substrate (#32132, manufactured by Thermo Fisher Scientific, Inc.) for 1 minute.

The PVDF membrane was photographed, and an amount of 15-PGDH protein was quantified based on density of staining by Image J software.

(5) Coomassie brilliant blue staining

Using the cryotube obtained in the above "(1) Specimen collection", the same operation as in the above "(3) SDS-PAGE" was performed to obtain an electrophoresed gel.

The gel after electrophoresis was immersed in Coomassie brilliant blue R-250 staining solution (Coomassie[R] blue R250, C.I.42660 (#6104-59-2, manufactured by Nacalai Tesque, Inc.)). Thereafter, staining was performed at room temperature while slowly shaking for 1 hour.

Next, the gel was immersed in a Destain solution (aqueous solution containing 7 wt% acetic acid and 5 wt% methanol) at 25°C for 16 hours to bleach the gel.

Visible bands on the gel were photographed, and an amount of total protein was quantified by Image J software.

Relative fold expression (rel to cal) was calculated based on the quantitative value of 15-PGDH and the quantitative value of total protein.

The relative fold expression can be calculated by the following formula (1). The results are shown in Table 7.

$$[\text{rel to call}] = 1000 \times [\text{quantitative value of 15- PGDH}] / [\text{quantitative value of total protein}] \qquad (1)$$

[Table 7]

| Subject | Success or failure of induction of labor | Quantitative value of 15-PGDH | Quantitative value of total protein | rel to cal |
|---|---|---|---|---|
| 1-27 | Success | 259896 | 204405090 | 1.27 |
| 1-28 | Success | 1395982 | 206894879 | 6.75 |
| 1-29 | Success | 1053748 | 202675796 | 5.20 |
| 1-30 | Success | 571869 | 208037727 | 2.75 |
| 1-31 | Success | 1646775 | 211633067 | 7.78 |
| 1-32 | Success | 788821 | 211780071 | 3.72 |
| 1-33 | Success | 681285 | 206502476 | 3.30 |
| 1-34 | Failure | 959425 | 212658843 | 4.51 |
| 1-35 | Failure | 1772781 | 208267388 | 8.51 |
| 1-36 | Failure | 710091 | 200126959 | 3.55 |
| 1-37 | Failure | 2905705 | 214581096 | 13.54 |
| 1-38 | Failure | 4523117 | 205469438 | 22.01 |
| 1-39 | Failure | 2700196 | 208449337 | 12.95 |
| 1-40 | Failure | 1968507 | 205069143 | 9.60 |
| 1-41 | Failure | 2200164 | 216594911 | 10.16 |
| 1-42 | Failure | 722697 | 200220760 | 3.61 |
| 1-43 | Failure | 546510 | 197420821 | 2.77 |
| 1-44 | Failure | 2116630 | 190950364 | 11.08 |

Regarding the "relative fold expression", the obtained data (18 points) were divided into a labor-induction success group (7 points) and a labor-induction failure group (11 points), and a P value when a null hypothesis "there is no difference in quantitative values of 15-PGDH between the labor-induction success group and the labor-induction failure group" was set was calculated, and the P value was 0.048.

That is, the relative fold expression was shown to be significantly lower in the labor-induction success group than that in the labor-induction failure group.

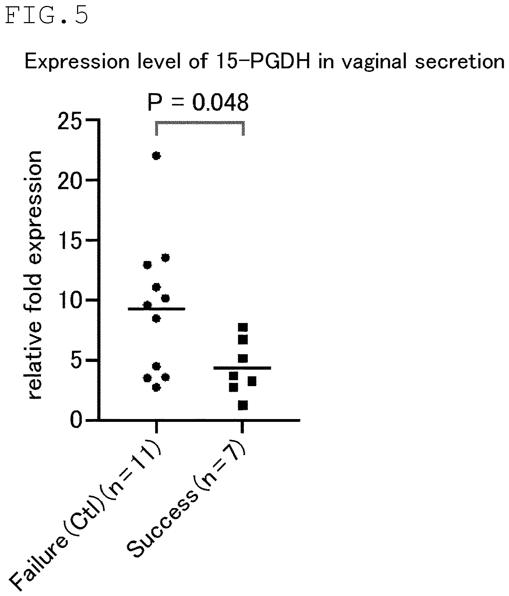

A distribution of values of relative fold expression in the labor-induction success group and the labor-induction failure group is shown in FIG. 5.

FIG. 5 is a diagram showing a distribution of values of relative fold expression using quantitative values of 15-PGDH in the labor-induction success group and the labor-induction failure group.

<Determination by Bishop score>

For the same subjects as in the above <Quantification of mRNA of 15-PGDH by quantitative PCR>, after collecting a vaginal secretion and before starting use of the oxytocin preparation, cervical ripening determination (Bishop score) by skilled obstetricians (6 obstetricians) was performed, scores were calculated according to the criteria in the following Table 8, and an average value of the scores by the 6 obstetricians was calculated. The results are shown in Table 9.

[Table 8]

|  | Score | | | |
|---|---|---|---|---|
|  | 0 | 1 | 2 | 3 |
| Effacement (%) | 0-30 | 40-50 | 60-70 | >80 |
| Station (-3 to +3) | -3 | -2 | -1 or 0 | +1 |
| Cervical Consistency | Firm | Middle | Soft | - |
| Dilatation (cm) | Closed | 1-2 | 3-4 | >=5 |
| Cervical Position | Posterior | Middle | Anterior | - |

[Table 9]

| Subject | Success or failure of induction of labor | Bishop score |
|---|---|---|
| 1-1 | Success | 0 |
| 1-2 | Success | 1 |
| 1-3 | Success | 0 |
| 1-4 | Success | 2 |
| 1-5 | Success | 3 |
| 1-6 | Success | 4 |
| 1-7 | Success | 3 |
| 1-8 | Success | 2 |
| 1-9 | Success | 3 |
| 1-10 | Success | 0 |
| 1-11 | Success | 5 |
| 1-12 | Success | 0 |
| 1-13 | Success | 3 |
| 1-14 | Success | 2 |
| 1-15 | Success | 4 |

(continued)

| Subject | Success or failure of induction of labor | Bishop score |
|---------|------------------------------------------|--------------|
| 1-16 | Success | 3 |
| 1-17 | Failure | 1 |
| 1-18 | Failure | 3 |
| 1-19 | Failure | 2 |
| 1-20 | Failure | 0 |
| 1-21 | Failure | 2 |
| 1-22 | Failure | 1 |
| 1-23 | Failure | 3 |
| 1-24 | Failure | 6 |
| 1-25 | Failure | 0 |
| 1-26 | Failure | 5 |

The obtained data (26 points) were divided into a labor-induction success group (16 points) and a labor-induction failure group (10 points), and a P value when a null hypothesis "there is no difference in Bishop score between the labor-induction success group and the labor-induction failure group" was set was calculated, and the P value was 0.88.

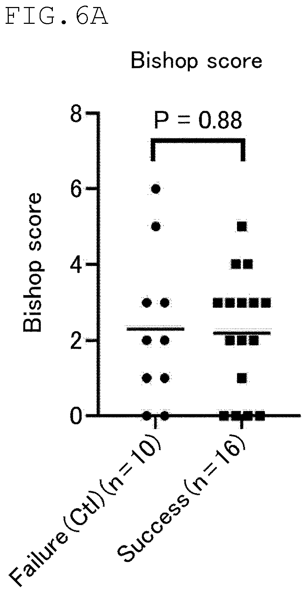

The results are shown in FIG. 6A.

FIG. 6A is a diagram showing a distribution of values of Bishop score in the labor-induction success group and the labor-induction failure group.

Only data of subject 1-7 were excluded, and a P value was calculated by the same method as described above. As a result, the P value was 0.82.

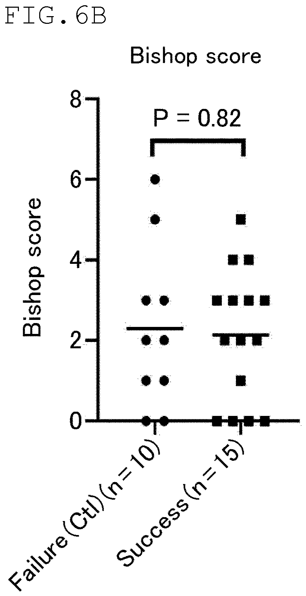

The results are shown in FIG. 6B.

FIG. 6B is a diagram showing a distribution of values of Bishop score in the labor-induction success group (excluding subject 1-7) and the labor-induction failure group.

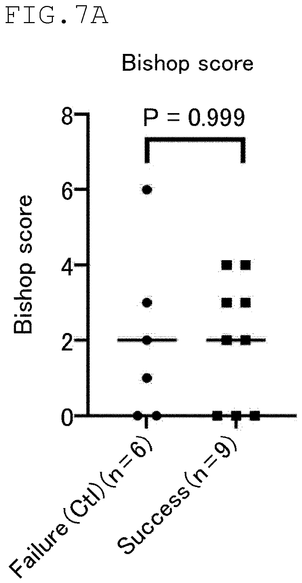

Among the obtained data, using data of only primiparous women [subjects 1-3, 1-4, 1-6, 1-7, 1-10, 1-12, 1-13, 1-14, 1-15, 1-18, 1-20, 1-21, 1-22, 1-24, and 1-25 in Table 9 (labor-induction success group (9 points) and labor-induction failure group (6 points))], a P value was calculated by the same method as described above, and the P value was 0.999. The results are shown in FIG. 7A.

FIG. 7A is a diagram showing a distribution of values of Bishop score in the labor-induction success group and the labor-induction failure group in subjects of only primiparous women.

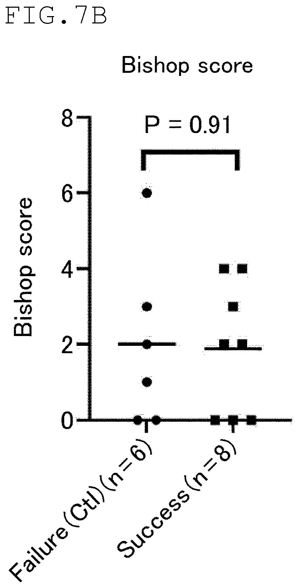

Only data of subject 1-7 were excluded, and a P value was calculated by the same method as described above. As a result, the P value was 0.91. The results are shown in FIG. 7B.

FIG. 7B is a diagram showing a distribution of values of Bishop score in the labor-induction success group (excluding subject 1-7) and the labor-induction failure group in subjects of only primiparous women.

The P value calculated in the above <Quantification of mRNA of 15-PGDH by quantitative PCR in women in the third trimester> and <Testing method including step of quantifying 15-PGDH in women in the third trimester (1)> is a significantly lower value when compared with the P value calculated in <Determination by Bishop score>.

Since the calculated P value was less than 0.05, the null hypothesis was confirmed to be rejected.

Therefore, it was found that success or failure of induction of labor can be predicted with high accuracy by using the data obtained by the above <Quantification of mRNA of 15-PGDH by quantitative PCR in women in the third trimester> and <Testing method including step of quantifying 15-PGDH in women in the third trimester (1)>.

Furthermore, when subjects are limited to only primiparous women, the P value becomes a lower value, and thus it was found that success or failure of induction of labor can be predicted with high accuracy.

Furthermore, since these methods are testing methods using a vaginal secretion as a specimen, they have an advantage of being non-invasive and less burdensome to a subject.

<Quantification of 15-PGDH using anti-15-PGDH antibody in women in the third trimester (2)>

25 women in the third trimester (25 subjects excluding subject 1-7 from 26 subjects in <Quantification of mRNA of 15-PGDH by quantitative PCR in women in the third trimester>) were taken as subjects, and a vaginal secretion was collected using a sterilized cotton swab at the time of gynecological examination using a speculum, and a vaginal secretion (including peeled epithelial tissue of cervix and vaginal squamous epithelial tissue) was collected.

Next, the cotton swab to which the vaginal secretion was attached was put into a cryotube containing 1.5 mL of PBS and stirred well to extract the vaginal secretion. Thereafter, the content of the cryotube was equally divided into two cryotubes (0.75 mL per cryotube), and stored at - 80°C until use for testing.

Using one of the two cryotubes, a total protein concentration in the specimen was measured by BCA assay using Pierce BCA Protein Assay Kit (#23227, manufactured by Thermo Fisher Scientific, Inc.). The obtained total protein concentration was divided by 1 mg/mL, and the reciprocal thereof was used as a correction coefficient.

Using one of the two cryotubes, centrifugation was performed under conditions of $12000 \times G$, 10 minutes, and 4°C to obtain a pellet.

The obtained pellet was dissolved in 375 µL of a RIPA buffer containing a protease inhibitor (Complete mini) [10 mL of a RIPA buffer (solution prepared by dissolving 8.766 g of NaCl, 10 mL of NP40, 5 g of sodium deoxycholate, 1 g of sodium dodecyl sulfate, and 6.055 g of trishydroxymethylaminomethane in distilled water to make a total amount of 1 L, and adjusting the pH to 7.4) and 1 tablet of Complete mini (Roche, #11836153001)] to produce a protein solution, and the solution was allowed to stand at 4°C for 10 minutes.

For the protein solution, a concentration of 15-PGDH was measured in the same manner as the method described in the above <Method for measuring concentration of 15-PGDH using solid-phase carrier reagent (A1) and labeling reagent (B1)>.

The measurement result is shown in Table 10.

In Table 10, the 15-PGDH correction value is a value obtained by multiplying a concentration of 15-PGDH of each specimen by the correction coefficient.

[Table 10]

| Subject | Success or failure of induction of labor | Correction coefficient | 15-PGDH concentration (pg/mL) | 15-PGDH correction value (pg/mL) |
|---|---|---|---|---|
| 1-1 | Success | 0.675 | 569 | 384 |
| 1-2 | Success | 0.821 | 194 | 159 |
| 1-3 | Success | 0.768 | 154 | 118 |
| 1-4 | Success | 0.289 | 118 | 34 |
| 1-5 | Success | 0.733 | 88 | 65 |
| 1-6 | Success | 0.416 | 160 | 66 |
| 1-8 | Success | 0.686 | 571 | 392 |
| 1-9 | Success | 0.424 | 127 | 54 |
| 1-10 | Success | 2.122 | 813 | 1725 |
| 1-11 | Success | 2.923 | 138 | 403 |
| 1-12 | Success | 0.324 | 15 | 5 |
| 1-13 | Success | 1.741 | 249 | 434 |
| 1-14 | Success | 2.177 | 27 | 59 |
| 1-15 | Success | 0.736 | 53 | 39 |
| 1-16 | Success | 3.029 | 36 | 109 |
| 1-17 | Failure | 0.625 | 233 | 146 |
| 1-18 | Failure | 0.567 | 1362 | 772 |
| 1-19 | Failure | 0.778 | 506 | 394 |
| 1-20 | Failure | 0.771 | 610 | 470 |
| 1-21 | Failure | 2.503 | 916 | 2293 |
| 1-22 | Failure | 1.520 | 678 | 1031 |

(continued)

| Subject | Success or failure of induction of labor | Correction coefficient | 15-PGDH concentration (pg/mL) | 15-PGDH correction value (pg/mL) |
|---|---|---|---|---|
| 1-23 | Failure | 0.944 | 131 | 124 |
| 1-24 | Failure | 1.451 | 167 | 242 |
| 1-25 | Failure | 1.548 | 1642 | 2542 |
| 1-26 | Failure | 0.965 | 579 | 559 |

Regarding quantitative values of 15-PGDH, respective subjects were divided into a labor-induction success group (15 points) and a labor-induction failure group (10 points), and a P value when a null hypothesis "there is no difference in quantitative values of 15-PGDH between the labor-induction success group and the labor-induction failure group" was set was calculated, and the P value was 0.0050.

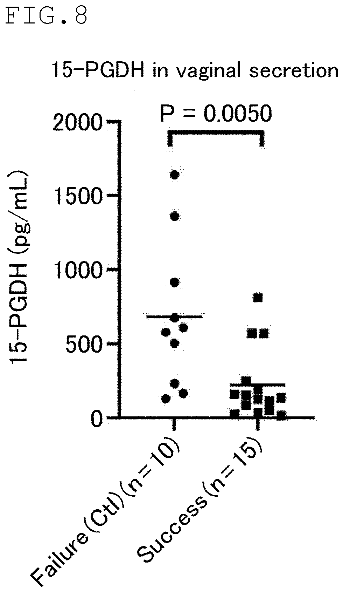

The results are shown in FIG. 8.

FIG. 8 is a diagram showing a distribution of quantitative values of 15-PGDH in the labor-induction success group and the labor-induction failure group.

With "being in a state in which induction of labor is successful" as a definition of 15-PGDH positivity, sensitivity, specificity, a matching rate with clinical practice (accuracy), a positive predictive value, and a negative predictive value when a cut-off value was set to 20 to 1500 pg/mL were calculated. The results are shown in Table 11.

[Table 11]

| Cut-off value (15-PGDH concentration) (pg/mL) | Sensitivity | Specificity | Matching rate with clinical practice | Positive predictive value | Negative predictive value |
|---|---|---|---|---|---|
| 20 | 7% | 100% | 44% | 100% | 42% |
| 30 | 13% | 100% | 48% | 100% | 43% |
| 40 | 20% | 100% | 52% | 100% | 45% |
| 50 | 20% | 100% | 52% | 100% | 45% |
| 70 | 27% | 100% | 56% | 100% | 48% |
| 90 | 33% | 100% | 60% | 100% | 50% |
| 100 | 33% | 100% | 60% | 100% | 50% |
| 150 | 53% | 90% | 68% | 89% | 56% |
| 200 | 73% | 80% | 76% | 85% | 67% |
| 250 | 80% | 70% | 76% | 80% | 70% |
| 300 | 80% | 70% | 76% | 80% | 70% |
| 350 | 80% | 70% | 76% | 80% | 70% |
| 400 | 80% | 70% | 76% | 80% | 70% |
| 450 | 80% | 70% | 76% | 80% | 70% |
| 500 | 80% | 70% | 76% | 80% | 70% |
| 550 | 80% | 60% | 72% | 75% | 67% |
| 600 | 93% | 50% | 76% | 74% | 83% |
| 650 | 93% | 40% | 72% | 70% | 80% |
| 700 | 93% | 30% | 68% | 67% | 75% |
| 750 | 93% | 30% | 68% | 67% | 75% |
| 800 | 93% | 30% | 68% | 67% | 75% |

(continued)

| Cut-off value (15-PGDH concentration) (pg/mL) | Sens itivity | Specificity | Matching rate with clinical practice | Pos itive predictive value | Negative predictive value |
|---|---|---|---|---|---|
| 850 | 100% | 30% | 72% | 68% | 100% |
| 900 | 100% | 30% | 72% | 68% | 100% |
| 950 | 100% | 20% | 68% | 65% | 100% |
| 1000 | 100% | 20% | 68% | 65% | 100% |
| 1200 | 100% | 20% | 68% | 65% | 100% |
| 1500 | 100% | 10% | 64% | 63% | 100% |

When 15-PGDH positivity is determined under the above conditions, the cut-off value is preferably set to any value in a range of 10 to 2000 pg/mL, more preferably set to any value in a range of 20 to 1500 pg/mL, still more preferably set to any value in a range of 40 to 900 pg/mL, particularly preferably set to any value in a range of 40 to 600 pg/mL.

When the cut-off value is set to any value in a range of 250 to 500 pg/mL, the sensitivity is 80%, the specificity is 70%, the matching rate with clinical practice (accuracy) is 76%, the positive predictive value is 80%, and the negative predictive value is 70%.

From the viewpoint of sensitivity, the cut-off value is preferably set to any value in a range of 150 to 2500 pg/mL, more preferably set to any value in a range of 200 to 2500 pg/mL, still more preferably set to any value in a range of 200 to 1500 pg/mL.

From the viewpoint of specificity, the cut-off value is preferably set to any value in a range of 10 to 600 pg/mL, more preferably set to any value in a range of 10 to 500 pg/mL, still more preferably set to any value in a range of 20 to 500 pg/mL.

From the viewpoint of matching rate with clinical practice (accuracy), the cut-off value is preferably set to any value in a range of 40 to 2500 pg/mL, more preferably set to any value in a range of 150 to 1200 pg/mL.

From the viewpoint of positive predictive value, the cut-off value is preferably set to any value in a range of 20 to 2500 pg/mL, more preferably set to any value in a range of 20 to 650 pg/mL.

From the viewpoint of negative predictive value, the cut-off value is preferably set to any value in a range of 90 to 1500 pg/mL, more preferably set to any value in a range of 200 to 1500 pg/mL.

When the cut-off value is set to any value of 200 pg/mL or less, it is possible to prevent oversight of failure of induction of labor with high probability.

When the cut-off value is set to any value of 850 pg/mL or more, it is possible to prevent oversight of success of induction of labor with high probability.

Since a preferable cut-off value varies depending on measurement conditions, the cut-off value is preferably appropriately set.

Among the obtained data, using data of only primiparous women [subjects 1-3, 1-4, 1-6, 1-10, 1-12, 1-13, 1-14, 1-15, 1-18, 1-20, 1-21, 1-22, 1-24, and 1-25 in Table 10 (labor-induction success group (8 points) and labor-induction failure group (6 points))], a P value was calculated by the same method as described above, and the P value was 0.0071.

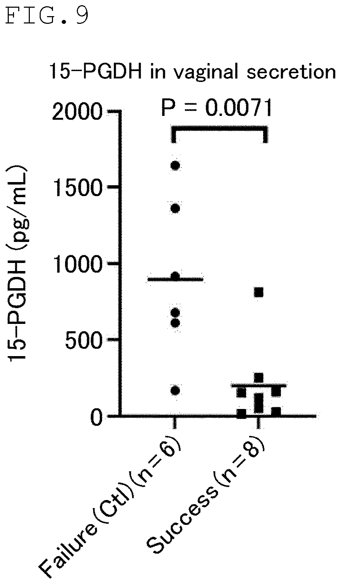

The results are shown in FIG. 9.

FIG. 9 is a diagram showing a distribution of quantitative values of 15-PGDH in the labor-induction success group and the labor-induction failure group in subjects of only primiparous women.

With "being in a state in which induction of labor is successful" as a definition of 15-PGDH positivity, sensitivity, specificity, a matching rate with clinical practice (accuracy), a positive predictive value, and a negative predictive value when a cut-off value was set to 20 to 1500 pg/mL were calculated. The results are shown in Table 12.

[Table 12]

| Cut-off value (15-PGDH concentration) (pg/mL) | Sensitivity | Specificity | Matching rate with clinical practice | Positive predictive value | Negative predictive value |
|---|---|---|---|---|---|
| 20 | 13% | 100% | 50% | 100% | 46% |
| 30 | 25% | 100% | 57% | 100% | 50% |
| 40 | 25% | 100% | 57% | 100% | 50% |

(continued)

| Cut-off value (15-PGDH concentration) (pg/mL) | Sensitivity | Specificity | Matching rate with clinical practice | Positive predictive value | Negative predictive value |
|---|---|---|---|---|---|
| 50 | 25% | 100% | 57% | 100% | 50% |
| 70 | 38% | 100% | 64% | 100% | 55% |
| 90 | 38% | 100% | 64% | 100% | 55% |
| 100 | 38% | 100% | 64% | 100% | 55% |
| 150 | 50% | 100% | 71% | 100% | 60% |
| 200 | 75% | 83% | 79% | 86% | 71% |
| 250 | 88% | 83% | 86% | 88% | 83% |
| 300 | 88% | 83% | 86% | 88% | 83% |
| 350 | 88% | 83% | 86% | 88% | 83% |
| 400 | 88% | 83% | 86% | 88% | 83% |
| 450 | 88% | 83% | 86% | 88% | 83% |
| 500 | 88% | 83% | 86% | 88% | 83% |
| 550 | 88% | 83% | 86% | 88% | 83% |
| 600 | 88% | 83% | 86% | 88% | 83% |
| 650 | 88% | 67% | 79% | 78% | 80% |
| 700 | 88% | 50% | 71% | 70% | 75% |
| 750 | 88% | 50% | 71% | 70% | 75% |
| 800 | 88% | 50% | 71% | 70% | 75% |
| 850 | 100% | 50% | 79% | 73% | 100% |
| 900 | 100% | 50% | 79% | 73% | 100% |
| 950 | 100% | 33% | 71% | 67% | 100% |
| 1000 | 100% | 33% | 71% | 67% | 100% |
| 1200 | 100% | 33% | 71% | 67% | 100% |
| 1500 | 100% | 17% | 64% | 62% | 100% |

When 15-PGDH positivity is determined under the above conditions, the cut-off value is preferably set to any value in a range of 10 to 2000 pg/mL, more preferably set to any value in a range of 20 to 1500 pg/mL, still more preferably set to any value in a range of 30 to 1200 pg/mL.

When the cut-off value is set to any value in a range of 250 to 600 pg/mL, the sensitivity is 88%, the specificity is 83%, the matching rate with clinical practice (accuracy) is 86%, the positive predictive value is 88%, and the negative predictive value is 83%.

From the viewpoint of sensitivity, the cut-off value is preferably set to any value in a range of 150 to 2500 pg/mL, more preferably set to any value in a range of 200 to 2500 pg/mL, still more preferably set to any value in a range of 200 to 1500 pg/mL.

From the viewpoint of specificity, the cut-off value is preferably set to any value in a range of 10 to 900 pg/mL, more preferably set to any value in a range of 10 to 600 pg/mL, still more preferably set to any value in a range of 20 to 600 pg/mL.

From the viewpoint of matching rate with clinical practice (accuracy), the cut-off value is preferably set to any value in a range of 20 to 2500 pg/mL, more preferably set to any value in a range of 150 to 1200 pg/mL.

From the viewpoint of positive predictive value, the cut-off value is preferably set to any value in a range of 20 to 2500 pg/mL, more preferably set to any value in a range of 20 to 900 pg/mL.

From the viewpoint of negative predictive value, the cut-off value is preferably set to any value in a range of 30 to 1500 pg/mL, more preferably set to any value in a range of 200 to 1500 pg/mL.

When the cut-off value is set to any value of 600 pg/mL or less, it is possible to prevent oversight of failure of induction of labor with high probability.

When the cut-off value is set to any value of 850 pg/mL or more, it is possible to prevent oversight of success of induction of labor with high probability.

Since a preferable cut-off value varies depending on measurement conditions, the cut-off value is preferably appropriately set.

When Table 11 and Table 12 are compared, a maximum value of the matching rate with clinical practice is higher when the data of only primiparous women are used. Therefore, the above method is useful for predicting success or failure of induction of labor in primiparous women.

Regarding 15-PGDH correction values, respective subjects were divided into a labor-induction success group (15 points) and a labor-induction failure group (10 points), and a P value when a null hypothesis "there is no difference in 15-PGDH correction values between the labor-induction success group and the labor-induction failure group" was set was calculated, and the P value was 0.034.

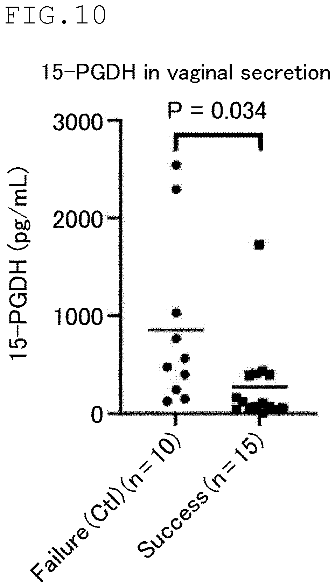

The results are shown in FIG. 10.

FIG. 10 is a diagram showing a distribution of 15-PGDH correction values in the labor-induction success group and the labor-induction failure group.

With "being in a state in which induction of labor is successful" as a definition of 15-PGDH positivity, sensitivity, specificity, a matching rate with clinical practice (accuracy), a positive predictive value, and a negative predictive value when a cut-off value was set to 20 to 2500 pg/mL were calculated. The results are shown in Table 13.

[Table 13]

| Cut-off value (15-PGDH correction value) (pg/mL) | Sensitivity | Specificity | Matching rate with clinical practice | Positive predictive value | Negative predictive value |
|---|---|---|---|---|---|
| 20 | 7% | 100% | 44% | 100% | 42% |
| 30 | 7% | 100% | 44% | 100% | 42% |
| 40 | 20% | 100% | 52% | 100% | 45% |
| 50 | 20% | 100% | 52% | 100% | 45% |
| 70 | 47% | 100% | 68% | 100% | 56% |
| 90 | 47% | 100% | 68% | 100% | 56% |
| 100 | 47% | 100% | 68% | 100% | 56% |
| 150 | 60% | 80% | 68% | 82% | 57% |
| 200 | 67% | 80% | 72% | 83% | 62% |
| 250 | 67% | 70% | 68% | 77% | 58% |
| 300 | 67% | 70% | 68% | 77% | 58% |
| 350 | 67% | 70% | 68% | 77% | 58% |
| 400 | 80% | 60% | 72% | 75% | 67% |
| 450 | 93% | 60% | 80% | 78% | 86% |
| 500 | 93% | 50% | 76% | 74% | 83% |
| 550 | 93% | 50% | 76% | 74% | 83% |
| 600 | 93% | 40% | 72% | 70% | 80% |
| 650 | 93% | 40% | 72% | 70% | 80% |
| 700 | 93% | 40% | 72% | 70% | 80% |
| 750 | 93% | 40% | 72% | 70% | 80% |
| 800 | 93% | 30% | 68% | 67% | 75% |
| 850 | 93% | 30% | 68% | 67% | 75% |

(continued)

| Cut-off value (15-PGDH correction value) (pg/mL) | Sensitivity | Specificity | Matching rate with clinical practice | Positive predictive value | Negative predictive value |
|---|---|---|---|---|---|
| 900 | 93% | 30% | 68% | 67% | 75% |
| 950 | 93% | 30% | 68% | 67% | 75% |
| 1000 | 93% | 30% | 68% | 67% | 75% |
| 1200 | 93% | 20% | 64% | 64% | 67% |
| 1500 | 93% | 20% | 64% | 64% | 67% |
| 1800 | 100% | 20% | 68% | 65% | 100% |
| 2500 | 100% | 10% | 64% | 63% | 100% |

When 15-PGDH positivity is determined under the above conditions, the cut-off value is preferably set to any value in a range of 10 to 3000 pg/mL, more preferably set to any value in a range of 10 to 2500 pg/mL, still more preferably set to any value in a range of 40 to 1000 pg/mL, particularly preferably set to any value in a range of 40 to 450 pg/mL.

When the cut-off value is set to any value in a range of 400 to 500 pg/mL, the sensitivity is 80 to 93%, the specificity is 50 to 60%, the matching rate with clinical practice (accuracy) is 72 to 80%, the positive predictive value is 74 to 78%, and the negative predictive value is 67 to 86%.

From the viewpoint of sensitivity, the cut-off value is preferably set to any value in a range of 150 to 3000 pg/mL, more preferably set to any value in a range of 400 to 3000 pg/mL, still more preferably set to any value in a range of 400 to 2500 pg/mL.

From the viewpoint of specificity, the cut-off value is preferably set to any value in a range of 10 to 550 pg/mL, more preferably set to any value in a range of 10 to 350 pg/mL, still more preferably set to any value in a range of 20 to 350 pg/mL.

From the viewpoint of matching rate with clinical practice (accuracy), the cut-off value is preferably set to any value in a range of 40 to 3000 pg/mL, more preferably set to any value in a range of 70 to 1000 pg/mL.

From the viewpoint of positive predictive value, the cut-off value is preferably set to any value in a range of 10 to 3000 pg/mL, more preferably set to any value in a range of 10 to 750 pg/mL, still more preferably set to any value in a range of 20 to 750 pg/mL.

From the viewpoint of negative predictive value, the cut-off value is preferably set to any value in a range of 70 to 2500 pg/mL, more preferably set to any value in a range of 400 to 2500 pg/mL.

When the cut-off value is set to any value of 200 pg/mL or less, it is possible to prevent oversight of failure of induction of labor with high probability.

When the cut-off value is set to any value of 1800 pg/mL or more, it is possible to prevent oversight of success of induction of labor with high probability.

Since a preferable cut-off value varies depending on measurement conditions, the cut-off value is preferably appropriately set.

Among the obtained data, using data of only primiparous women [subjects 1-3, 1-4, 1-6, 1-10, 1-12, 1-13, 1-14, 1-15, 1-18, 1-20, 1-21, 1-22, 1-24, and 1-25 in Table 13 (labor-induction success group (8 points) and labor-induction failure group (6 points))], a P value was calculated by the same method as described above, and the P value was 0.048.

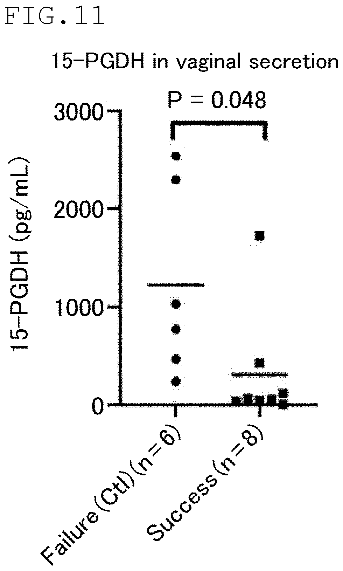

The results are shown in FIG. 11.

FIG. 11 is a diagram showing a distribution of 15-PGDH correction values in the labor-induction success group and the labor-induction failure group in subjects of only primiparous women.

With "being in a state in which induction of labor is successful" as a definition of 15-PGDH positivity, sensitivity, specificity, a matching rate with clinical practice (accuracy), a positive predictive value, and a negative predictive value when a cut-off value was set to 20 to 2500 pg/mL were calculated. The results are shown in Table 14.

[Table 14]

| Cut-off value (15-PGDH correction value) (pg/mL) | Sensitivity | Specificity | Matching rate with clinical practice | Positive predictive value | Negative predictive value |
|---|---|---|---|---|---|
| 20 | 13% | 100% | 50% | 100% | 46% |

(continued)

| Cut-off value (15-PGDH correction value) (pg/mL) | Sensitivity | Specificity | Matching rate with clinical practice | Positive predictive value | Negative predictive value |
|---|---|---|---|---|---|
| 30 | 13% | 100% | 50% | 100% | 46% |
| 40 | 38% | 100% | 64% | 100% | 55% |
| 50 | 38% | 100% | 64% | 100% | 55% |
| 70 | 63% | 100% | 79% | 100% | 67% |
| 90 | 63% | 100% | 79% | 100% | 67% |
| 100 | 63% | 100% | 79% | 100% | 67% |
| 150 | 75% | 100% | 86% | 100% | 75% |
| 200 | 75% | 100% | 86% | 100% | 75% |
| 250 | 75% | 83% | 79% | 86% | 71% |
| 300 | 75% | 83% | 79% | 86% | 71% |
| 350 | 75% | 83% | 79% | 86% | 71% |
| 400 | 75% | 83% | 79% | 86% | 71% |
| 450 | 88% | 83% | 86% | 88% | 83% |
| 500 | 88% | 67% | 79% | 78% | 80% |
| 550 | 88% | 67% | 79% | 78% | 80% |
| 600 | 88% | 67% | 79% | 78% | 80% |
| 650 | 88% | 67% | 79% | 78% | 80% |
| 700 | 88% | 67% | 79% | 78% | 80% |
| 750 | 88% | 67% | 79% | 78% | 80% |
| 800 | 88% | 50% | 71% | 70% | 75% |
| 850 | 88% | 50% | 71% | 70% | 75% |
| 900 | 88% | 50% | 71% | 70% | 75% |
| 950 | 88% | 50% | 71% | 70% | 75% |
| 1000 | 88% | 50% | 71% | 70% | 75% |
| 1200 | 88% | 33% | 64% | 64% | 67% |
| 1500 | 88% | 33% | 64% | 64% | 67% |
| 1800 | 100% | 33% | 71% | 67% | 100% |
| 2500 | 100% | 17% | 64% | 62% | 100% |

When 15-PGDH positivity is determined under the above conditions, the cut-off value is preferably set to any value in a range of 10 to 3000 pg/mL, more preferably set to any value in a range of 10 to 2500 pg/mL, still more preferably set to any value in a range of 40 to 2000 pg/mL, particularly preferably set to any value in a range of 40 to 450 pg/mL.

When the cut-off value is set to any value in a range of 150 to 200 pg/mL, the sensitivity is 75%, the specificity is 100%, the matching rate with clinical practice (accuracy) is 86%, the positive predictive value is 100%, and the negative predictive value is 75%.

From the viewpoint of sensitivity, the cut-off value is preferably set to any value in a range of 70 to 3000 pg/mL, more preferably set to any value in a range of 150 to 3000 pg/mL, still more preferably set to any value in a range of 150 to 2500 pg/mL.

From the viewpoint of specificity, the cut-off value is preferably set to any value in a range of 10 to 1000 pg/mL, more preferably set to any value in a range of 10 to 450 pg/mL, still more preferably set to any value in a range of 20 to 450 pg/mL.

From the viewpoint of matching rate with clinical practice (accuracy), the cut-off value is preferably set to any value in a range of 10 to 3000 pg/mL, more preferably set to any value in a range of 10 to 1000 pg/mL, still more preferably set to any value in a range of 20 to 1000 pg/mL.

From the viewpoint of positive predictive value, the cut-off value is preferably set to any value in a range of 10 to 3000 pg/mL, more preferably set to any value in a range of 10 to 1000 pg/mL, still more preferably set to any value in a range of 20 to 1000 pg/mL.

From the viewpoint of negative predictive value, the cut-off value is preferably set to any value in a range of 40 to 2500 pg/mL, more preferably set to any value in a range of 70 to 2500 pg/mL.

When the cut-off value is set to any value of 450 pg/mL or less, it is possible to prevent oversight of failure of induction of labor with high probability.

When the cut-off value is set to any value of 1800 pg/mL or more, it is possible to prevent oversight of success of induction of labor with high probability.

Since a preferable cut-off value varies depending on measurement conditions, the cut-off value is preferably appropriately set.

<Correlation of method for measuring expression level of 15-PGDH>

Data of 25 women in the third trimester (25 women excluding subject 1-7) were plotted on a graph in which the horizontal axis was the value of relative fold expression calculated in the above <Quantification of mRNA of 15-PGDH by quantitative PCR in women in the third trimester> and the vertical axis was the quantitative value of 15-PGDH measured in the above <Quantification of 15-PGDH using anti-15-PGDH antibody in women in late pregnancy (2)>, and a correlation coefficient R was calculated. As a result, the value was 0.674.

The obtained data were plotted on a graph in which the horizontal axis was not changed and the vertical axis was the 15-PGDH correction value measured in the above <Quantification of 15-PGDH using anti-15-PGDH antibody in women in late pregnancy (2)>, and a correlation coefficient R was calculated. As a result, the value was 0.460.

Among the obtained data, data of only primiparous women were plotted on a graph in which the horizontal axis was the value of relative fold expression calculated in the above <Quantification of mRNA of 15-PGDH by quantitative PCR in women in late pregnancy> and the vertical axis was the quantitative value of 15-PGDH measured in the above <Quantification of 15-PGDH using anti-15-PGDH antibody in women in late pregnancy (2)>, and a correlation coefficient R was calculated. As a result, the value was 0.710.

The obtained data were plotted on a graph in which the horizontal axis was not changed and the vertical axis was the 15-PGDH correction value measured in the above <Quantification of 15-PGDH using anti-15-PGDH antibody in women in late pregnancy (2)>, and a correlation coefficient R was calculated. As a result, the value was 0.511.

<Quantification of mRNA of 15-PGDH in subjects who experienced preterm birth>

(1) Specimen collection

5 pregnant women with a history of cervical surgery (extensive cervical resection or conization) at 21 to 31 weeks of gestation were taken as subjects, and a vaginal secretion was collected using a sterilized cotton swab at the time of prenatal checkup using a speculum, and a vaginal secretion (including peeled epithelial tissue of cervix and vaginal squamous epithelial tissue) was collected.

2 of the 5 women subsequently delivered at 33 weeks of gestation (cases of preterm birth).

The other 4 women subsequently delivered at or after 37 weeks of gestation (cases of no preterm birth).

Next, the cotton swab to which the vaginal secretion was attached was put into a cryotube containing 1.5 mL of RNA later [RNA later Soln (#AM7021, manufactured by Invitrogen)] and stirred well to extract the vaginal secretion. Thereafter, the cryotube was stored at -80°C until use for testing.

Thereafter, the same operations as in "(2) Induction of labor" and "(3) Quantitative PCR" in the above <Quantification of mRNA of 15-PGDH by quantitative PCR> were performed, and relative fold expression (rel to cal) was calculated.

The results are shown in Table 15.

[Table 15]

| Subject | Case | Timing of specimen inoculation | Whether preterm birth occurred | | Expression level of m RNA (Ct) | | dCt | ddCt | rel to cal |
|---|---|---|---|---|---|---|---|---|---|
| | | | Number of weeks of delivery | | 15-PGDH | GAPDH | | | |
| 2-1 | Extensive cervical resection | 24 weeks of gestation | 33 weeks of gestation | Case of preterm birth | 25.63 | 21.39 | 4.24 | 4.78 | 0.04 |
| 2-2 | Extensive cervical resection | 21 weeks of gestation | 33 weeks of gestation | Case of preterm birth | 24.17 | 23.33 | 0.84 | 1.38 | 0.38 |
| 2-3 | Conization | 27 weeks of gestation | 39 weeks of gestation | Case of no preterm birth | 21.96 | 21.45 | 0.50 | 1.04 | 0.49 |
| 2-4 | Conization | 29 weeks of gestation | 41 weeks of gestation | Case of no preterm birth | 22.65 | 22.37 | 0.28 | 0.82 | 0.57 |
| 2-5 | Conization | 31 weeks of gestation | 37 weeks of gestation | Case of no preterm birth | 21.83 | 22.02 | -0.19 | 0.35 | 0.79 |

As shown in Table 15, it can be seen that the relative fold expression is remarkably decreased in the cases of preterm birth as compared with the cases of no preterm birth.

It was found that the expression level of 15-PGDH in a vaginal secretion is not only an index for success or failure of induction of labor but also an index for preterm birth, and preterm birth can be predicted.

<Quantification of 15-PGDH in subjects who experienced preterm birth>

(1) Specimen collection

30 pregnant women at 24 to 28 weeks of gestation were taken as subjects, and a vaginal secretion was collected using a sterilized cotton swab at the time of prenatal checkup using a speculum, and a vaginal secretion (including peeled epithelial tissue of cervix and vaginal squamous epithelial tissue) was collected.

6 of the 30 women subsequently delivered at 30 to 34 weeks of gestation (cases of preterm birth).

The other 24 women subsequently delivered at or after 37 weeks of gestation (cases of no preterm birth).

Next, the cotton swab to which the vaginal secretion was attached was put into a cryotube containing 1.5 mL of PBS and stirred well to extract the vaginal secretion. Thereafter, the content of the cryotube was equally divided into three cryotubes (0.50 mL per three cryotube), and stored at -80°C until use for testing.

An amount of total protein, a concentration of PGE2, and a concentration of 15-PGDH in the specimen were measured in the same manner as in (Measurement of total protein concentration), (Measurement of concentration of PGE2), and (Method for measuring concentration of 15-PGDH using solid-phase carrier reagent (A1) and labeling reagent (B1)) described in the above <Confirmation of behavior of expression level of 15-PGDH from pregnancy to delivery>. A reciprocal of a value obtained by dividing the obtained total protein concentration by 1 mg/mL was used as a correction coefficient, and the concentration of 15-PGDH was multiplied by the correction coefficient to calculate a 15-PGDH correction value.

A value obtained by dividing the concentration of 15-PGDH by the concentration of PGE2 was calculated.

For the concentration of 15-PGDH, the 15-PGDH correction value, and the value obtained by dividing the concentration of 15-PGDH by the concentration of PGE2, with "being in a state of preterm birth" as a definition of 15-PGDH positivity, sensitivity, specificity, a matching rate with clinical practice (accuracy), a positive predictive value, and a negative predictive value when a cut-off value was set as shown in Tables 16, 17, and 18 were calculated. The results are shown in Table 16, Table 17, and Table 18.

Table 16 is a table showing results when a cut-off value is set based on the concentration of 15-PGDH.

Table 17 is a table showing results when a cut-off value is set based on the 15-PGDH correction value.

Table 18 is a table showing results when a cut-off value is set based on the value obtained by dividing the concentration of 15-PGDH by the concentration of PGE2.

[Table 16]

| Cut-off value (15-PGDH concentration) (pg/mL) | Sensitivity | Specificity | Matching rate with clinical practice | Positive predictive value | Negative predictive value |
|---|---|---|---|---|---|
| 250 | 17% | 100% | 83% | 100% | 83% |
| 300 | 33% | 100% | 87% | 100% | 86% |
| 400 | 33% | 92% | 80% | 50% | 85% |
| 500 | 33% | 92% | 80% | 50% | 85% |
| 600 | 33% | 83% | 73% | 33% | 83% |
| 700 | 33% | 71% | 63% | 22% | 81% |
| 800 | 33% | 58% | 53% | 17% | 78% |
| 900 | 50% | 50% | 50% | 20% | 80% |
| 1000 | 50% | 42% | 43% | 18% | 77% |
| 1100 | 50% | 29% | 33% | 15% | 70% |
| 1200 | 50% | 25% | 30% | 14% | 67% |
| 1300 | 50% | 21% | 27% | 14% | 63% |
| 1400 | 67% | 17% | 27% | 17% | 67% |
| 1900 | 67% | 13% | 23% | 16% | 60% |
| 2000 | 83% | 13% | 27% | 19% | 75% |
| 2400 | 100% | 13% | 30% | 22% | 100% |
| 2600 | 100% | 8% | 27% | 21% | 100% |

[Table 17]

| Cut-off value (15-PGDH correction value) (pg/mL) | Sensitivity | Specificity | Matching rate with clinical practice | Positive predictive value | Negative predictive value |
|---|---|---|---|---|---|
| 100 | 17% | 100% | 83% | 100% | 83% |
| 200 | 17% | 88% | 73% | 25% | 81% |
| 300 | 17% | 58% | 50% | 9% | 74% |
| 330 | 33% | 54% | 50% | 15% | 76% |
| 340 | 67% | 54% | 57% | 27% | 87% |
| 360 | 83% | 54% | 60% | 31% | 93% |
| 400 | 83% | 50% | 57% | 29% | 92% |
| 500 | 83% | 38% | 47% | 25% | 90% |
| 600 | 83% | 21% | 33% | 21% | 83% |
| 700 | 83% | 8% | 23% | 19% | 67% |
| 750 | 83% | 8% | 23% | 19% | 67% |

[Table 18]

| Cut-off value (15-PGDH concentration/PGE2) | Sensitivity | Specificity | Matching rate with clinical practice | Positive predictive value | Negative predictive value |
|---|---|---|---|---|---|
| 1 | 17% | 92% | 77% | 33% | 81% |
| 3 | 33% | 71% | 63% | 22% | 81% |
| 5 | 33% | 58% | 53% | 17% | 78% |
| 10 | 50% | 50% | 50% | 20% | 80% |
| 15 | 67% | 38% | 43% | 21% | 82% |
| 20 | 67% | 38% | 43% | 21% | 82% |
| 30 | 67% | 33% | 40% | 20% | 80% |
| 40 | 67% | 29% | 37% | 19% | 78% |
| 50 | 67% | 21% | 30% | 17% | 71% |
| 60 | 67% | 17% | 27% | 17% | 67% |
| 70 | 67% | 13% | 23% | 16% | 60% |
| 80 | 83% | 13% | 27% | 19% | 75% |
| 90 | 83% | 13% | 27% | 19% | 75% |
| 100 | 83% | 8% | 23% | 19% | 67% |
| 250 | 83% | 4% | 20% | 18% | 50% |
| 400 | 100% | 4% | 23% | 21% | 100% |

When 15-PGDH positivity is determined under the above conditions, and a cut-off value is set based on the concentration of 15-PGDH, the cut-off value is preferably set to any value in a range of 10 to 3000 pg/mL, more preferably set to any value in a range of 20 to 2600 pg/mL, still more preferably set to any value in a range of 100 to 900 pg/mL.

When the cut-off value is set to any value in a range of 300 to 500 pg/mL, the sensitivity is 33%, the specificity is 92 to 100%, the matching rate with clinical practice (accuracy) is 80 to 87%, the positive predictive value is 50 to 100%, and the negative predictive value is 85 to 86%.

From the viewpoint of sensitivity, the cut-off value is preferably set to any value in a range of 250 to 3000 pg/mL, more preferably set to any value in a range of 300 to 3000 pg/mL, still more preferably set to any value in a range of 300 to 2600 pg/mL.

From the viewpoint of specificity, the cut-off value is preferably set to any value in a range of 10 to 900 pg/mL, more preferably set to any value in a range of 10 to 500 pg/mL, still more preferably set to any value in a range of 250 to 500 pg/mL.

From the viewpoint of matching rate with clinical practice (accuracy), the cut-off value is preferably set to any value in a range of 10 to 2400 pg/mL, more preferably set to any value in a range of 10 to 900 pg/mL, still more preferably set to any value in a range of 250 to 900 pg/mL.

From the viewpoint of positive predictive value, the cut-off value is preferably set to any value in a range of 250 to 3000 pg/mL, more preferably set to any value in a range of 250 to 600 pg/mL.

From the viewpoint of negative predictive value, the cut-off value is preferably set to any value in a range of 10 to 2600 pg/mL, more preferably set to any value in a range of 250 to 2600 pg/mL.

When the cut-off value is set to any value of 900 pg/mL or more, it is possible to prevent oversight of preterm birth with high probability.

When 15-PGDH positivity is determined under the above conditions, and a cut-off value is set based on the 15-PGDH correction value, the cut-off value is preferably set to any value in a range of 10 to 3000 pg/mL, more preferably set to any value in a range of 20 to 1500 pg/mL, still more preferably set to any value in a range of 100 to 750 pg/mL.

When the cut-off value is set to any value in a range of 100 to 200 pg/mL, the sensitivity is 17%, the specificity is 88 to 100%, the matching rate with clinical practice (accuracy) is 73 to 83%, the positive predictive value is 25 to 100%, and the negative predictive value is 81 to 83%.

From the viewpoint of sensitivity, the cut-off value is preferably set to any value in a range of 100 to 3000 pg/mL, more preferably set to any value in a range of 330 to 3000 pg/mL, still more preferably set to any value in a range of 330 to 750 pg/mL.

From the viewpoint of specificity, the cut-off value is preferably set to any value in a range of 10 to 500 pg/mL, more preferably set to any value in a range of 10 to 200 pg/mL, still more preferably set to any value in a range of 100 to 200 pg/mL.

From the viewpoint of matching rate with clinical practice (accuracy), the cut-off value is preferably set to any value in a range of 10 to 750 pg/mL, more preferably set to any value in a range of 10 to 500 pg/mL, still more preferably set to any value in a range of 100 to 500 pg/mL.

From the viewpoint of positive predictive value, the cut-off value is preferably set to any value in a range of 10 to 3000 pg/mL, more preferably set to any value in a range of 100 to 600 pg/mL.

From the viewpoint of negative predictive value, the cut-off value is preferably set to any value in a range of 10 to 2600 pg/mL, more preferably set to any value in a range of 10 to 750 pg/mL, still more preferably set to any value in a range of 100 to 750 pg/mL.

When the cut-off value is set to any value of 340 pg/mL or more, it is possible to prevent oversight of preterm birth with high probability.

When 15-PGDH positivity is determined under the above conditions, and a cut-off value is set based on the value obtained by dividing the concentration of 15-PGDH by the concentration of PGE2, the cut-off value is preferably set to any value in a range of 0.1 to 1000, more preferably set to any value in a range of 0.1 to 400, still more preferably set to any value in a range of 1 to 50.

When the cut-off value is set to any value in a range of 1 to 10, the sensitivity is 17 to 50%, the specificity is 50 to 92%, the matching rate with clinical practice (accuracy) is 50 to 77%, the positive predictive value is 17 to 33%, and the negative predictive value is 78 to 81%.

From the viewpoint of sensitivity, the cut-off value is preferably set to any value in a range of 1 to 1000, more preferably set to any value in a range of 3 to 1000, still more preferably set to any value in a range of 3 to 400.

From the viewpoint of specificity, the cut-off value is preferably set to any value in a range of 0.1 to 30, more preferably set to any value in a range of 0.1 to 10, still more preferably set to any value in a range of 1 to 10.

From the viewpoint of matching rate with clinical practice (accuracy), the cut-off value is preferably set to any value in a range of 0.1 to 50, more preferably set to any value in a range of 0.1 to 10, still more preferably set to any value in a range of 1 to 10.

From the viewpoint of positive predictive value, the cut-off value is preferably set to any value in a range of 0.1 to 1000, more preferably set to any value in a range of 0.1 to 30, still more preferably set to any value in a range of 1 to 30.

From the viewpoint of negative predictive value, the cut-off value is preferably set to any value in a range of 0.1 to 1000, more preferably set to any value in a range of 0.1 to 30, still more preferably set to any value in a range of 1 to 30.

When the cut-off value is set to any value of 80 or more, it is possible to prevent oversight of preterm birth with high probability.

Since a preferable cut-off value varies depending on measurement conditions, the cut-off value is preferably appropriately set.

<Production of ELISA reagent>

An anti-15-PGDH antibody that can be used in the testing method of the present invention (that can be supported on a solid-phase carrier, and can also be used as a raw material of a labeled anti-15-PGDH antibody) was produced by the following method.

(Anti-15-PGDH antibody)

Production of the anti-15-PGDH antibody was performed according to a conventional method of producing an antibody by immunization of a mouse and cell fusion.

That is, after immunizing a mouse with 15-PGDH (protein to which a 6-His tag is added at the C-terminal of a protein having an amino acid sequence of SEQ ID NO: 1), splenocytes producing an antibody against 15-PGDH (anti-15-PGDH antibody) (antibody-producing cells) were collected from the mouse, and the splenocytes were subjected to cell fusion with myeloma cells for screening, thereby obtaining culture fluids (11 types) containing hybridoma producing an anti-15-PGDH monoclonal antibody [using a supernatant of one type of culture fluid among them (culture supernatant containing the anti-15-PGDH antibody), evaluation of the anti-15-PGDH antibody described later was performed].

Thereafter, the cultured hybridoma was intraperitoneally injected into the mouse, and 10 days later, ascites was collected, and the ascites was purified to obtain the anti-15-PGDH antibody.

Each reagent used for evaluation of the anti-15-PGDH antibody described later was prepared by the following method.

(10 × PBS (-))

2 g of potassium dihydrogen phosphate, 2 g of potassium chloride, 11.5 g of disodium hydrogen phosphate, and 80 g of sodium chloride were charged in a 1,000 mL volumetric flask. Thereafter, water was charged so that the volume of the solution was 1,000 mL, and uniformly mixed at 25°C to prepare "10× PBS(-)".

(1 × PBS (-))

The above "10× PBS(-)" was diluted 10 times with water to prepare "1× PBS(-)".

(PBS-T)

To the above "1× PBS(-)", 0.05 wt% Tween 20 (manufactured by Tokyo Chemical Industry Co., Ltd.) was added to prepare "PBS-T".

(Blocking Buf)

To the above "PBS-T", 0.2 wt% bovine serum albumin (manufactured by Boval Company) was added and mixed, and the mixture was filtered through a 0.45 um membrane filter to prepare "Blocking Buf".

(Labeling reagent containing POD-labeled anti-mouse IgG antibody)

Using an anti-mouse IgG antibody and horseradish-derived POD (manufactured by TOYOBO CO., LTD.), a POD-labeled anti-mouse IgG antibody was prepared by the method described in the literature (S. Yoshitake, M. Imagawa, E. Ishikawa, et al.; J. Biochem, Vol. 92, 1982, 1413-1424).
This was diluted 1000 times (by volume) or 3000 times (by volume) using the above Blocking Buf to prepare two types of labeling reagents, and the labeling reagents were stored in a refrigerator (2°C to 10°C).

(Preparation of specimen: specimen collected from placenta)

(1) A placenta tissue was collected in a 2 cm square from a placenta of a cesarean section case, and then washed with PBS.
The placenta tissue was rapidly frozen with liquid nitrogen, and then the tissue was crushed using a Bessman tissue pulverizer. The crushed tissue was transferred to a 50 mL Falcon tube.
Thereto was added 10 mL of PBS.
(2) The tube was well vortexed.
(3) The content of the tube was equalized at 4°C. Specifically, the crushed tissue dissolved in PBS was further crushed using a homogenizer (Polytron PT3100, manufactured by Polytron).
(4) The content of the tube was stirred at 4°C for 16 hours in a cold room.
(5) The tube was centrifuged at 4°C and 8000 G for 20 minutes.
(6) The supernatant (specimen) after centrifugation was transferred to a new 50 mL Falcon tube.
(7) The content of the Falcon tube was rapidly frozen with liquid nitrogen.
(8) The tube was stored in a freezer at -80°C.

(Preparation of specimen: specimen collected from amnion)

A specimen was obtained in the same manner except that the step of "(1)" was changed to the following step of "(1-1)" in the above (Preparation of specimen: specimen collected from placenta).
(1-1) From the placenta of a cesarean section case in the above (Preparation of specimen: specimen collected from placenta), fetal membranes were collected in a 5 cm square, washed once with PBS, and then an amnion was manually peeled off.
The amnion tissue was rapidly frozen with liquid nitrogen, and then the tissue was crushed using a Bessman tissue pulverizer. The crushed tissue was transferred to a 50 mL Falcon tube.
Thereto was added 10 mL of PBS.

(Preparation of specimen: specimen collected from chorion)

A specimen was obtained in the same manner except that the step of "(1-1)" was changed to the following step of "(1-2)" in the above (Preparation of specimen: specimen collected from amnion).

(1-2) For the fetal membranes (choriodecidua) after collecting the amnion in the above (Preparation of specimen: specimen collected from amnion), a chorion was collected in a 5 cm square on a glass plate using a slide glass.

The chorion tissue was rapidly frozen with liquid nitrogen, and then the tissue was crushed using a Bessman tissue pulverizer. The crushed tissue was transferred to a 50 mL Falcon tube.

Thereto was added 10 mL of PBS.

(Preparation of specimen: specimen collected from decidua)

A specimen was obtained in the same manner except that the step of "(1-2)" was changed to the following step of "(1-3)" in the above (Preparation of specimen: specimen collected from chorion).

(1-3) From the choriodecidua in the above (Preparation of specimen: specimen collected from chorion), the remaining tissue (decidua) after removal of the chorion was obtained at 5 cm square.

The decidua tissue was rapidly frozen with liquid nitrogen, and then the tissue was crushed using a Bessman tissue pulverizer. The crushed tissue was transferred to a 50 mL Falcon tube.

Thereto was added 10 mL of PBS.

It was confirmed by the methods described in (1) to (11) below that the testing method of the present invention can be used for the anti-15-PGDH antibody (culture supernatant containing the anti-15-PGDH antibody) obtained in the above (Anti-15-PGDH antibody).

(1) Solid-phasing

To an ELISA plate 96F H (manufactured by Sumitomo Bakelite Co., Ltd.), 100 μL each of a specimen of each site was added, and the plate was allowed to stand at 4°C for 21 hours. By this operation, 15-PGDH contained in the specimen of each site was immobilized on the ELISA plate.

(2) Specimen removal

The specimen supernatant added to the ELISA plate 96F H at the time of solid-phasing was removed.

(3) Blocking

350 μL of Blocking Buf was added to each well of the ELISA plate 96F H, and the plate was allowed to stand (25°C, protected from light) for 2 hours.

(4) Washing

Each well of the ELISA plate 96F H was washed with PBS-T 350 μL × 3 times.

(5) Culture supernatant (monoclonal antibody) reaction

The supernatant of the culture fluid containing the anti-15-PGDH antibody obtained in the above (Anti-15-PGDH antibody) was diluted 5 times with PBS-T, and 100 μL of the diluted supernatant was added to each well of the ELISA plate 96F H, and allowed to stand to react for 1 hour (25°C, protected from light).

By this operation, a complex of 15-PGDH contained in the specimen of each site and the anti-15-PGDH antibody was formed on the ELISA plate.

(6) Washing

Each well of the ELISA plate 96F H was washed with PBS-T 350 μL × 3 times.

(7) Secondary antibody reaction

100 μL of the labeling reagent (1000-fold diluted reagent or 3000-fold diluted reagent) prepared above was added to each well of the ELISA plate 96F H.

Subsequently, the mixture was allowed to stand to react in a 35°C incubator (low-temperature thermostat LTE-1000, manufactured by Tokyo Garasu Kikai Co., Ltd.) for 1 hour.

By this operation, a complex of 15-PGDH contained in the specimen of each site, the anti-15-PGDH antibody, and the anti-mouse IgG antibody was formed on the ELISA plate.

(8) Washing

Each well of the ELISA plate 96F H was washed with PBS-T 350 $\mu$L $\times$ 3 times.

(9) Color development

100 $\mu$L of a color developing solution (1 step Turbo TMB -ELISA Substrate Solution, manufactured by Thermo Fisher Scientific, Inc.) was added to each well of the ELISA plate 96F H, and allowed to stand to react for 5 minutes (25°C).

(10) Stop of color development reaction

100 $\mu$L of 2 M $H_2SO_4$ was added to each well of the ELISA plate 96F H to stop the color development reaction.

(11) Absorbance measurement

Absorbance at 450 nm was measured using an automated ELISA workstation DS2 (registered trademark) (Dynex Technologies Inc.).

The measurement was performed using two types of labeling reagents for each specimen of each site, and the measurement was performed twice for each specimen. The results are shown in Table 19.

[Table 19]

| Site of specimen | 1000-fold dilution | | 3000-fold dilution | |
|---|---|---|---|---|
| | n = 1 | n = 2 | n = 1 | n = 2 |
| Amnion | 0.47 | 0.49 | 0.33 | 0.32 |
| Placenta | 0.69 | 0.72 | 0.42 | 0.42 |
| Decidua | 1.95 | 1.95 | 1.08 | 1.07 |
| Chorion | 2.05 | 2.05 | 1.40 | 1.36 |

Also in measurement using the culture supernatant containing the anti-15-PGDH antibody, intensity of a signal increased in the order of amnion, placenta, decidua, and chorion as shown in Table 19.

This was similar to the results shown in FIG. 2.

From this result, the anti-15-PGDH antibody obtained by the operation in the above (Anti-15-PGDH antibody) was shown to be useful as an antibody to be supported on a solid-phase carrier used in the testing method of the present invention and as a raw material of a labeled anti-15-PGDH antibody.

The ELISA reagent produced using the anti-15-PGDH antibody is the testing reagent of the present invention.

In order to confirm that the anti-15-PGDH antibody obtained by the operation in the above (Anti-15-PGDH antibody) can be used for, for example, first non-competitive immunological measurement, a first anti-15-PGDH monoclonal antibody was used as a solid phase, and a second anti-15-PGDH antibody was used as a labeled antibody. Then, using the above "specimen collected from placenta", "specimen collected from amnion", "specimen collected from chorion", and "specimen collected from decidua", the amount of luminescence was confirmed. The results are shown in Table 20.

[Table 20]

| Site of specimen | Amount of luminescence of 15-PGDH (cps) |
|---|---|
| Amnion | 4,273 |
| Placenta | 47,360 |
| Decidua | 472,307 |

(continued)

| Site of specimen | Amount of luminescence of 15-PGDH (cps) |
|---|---|
| Chorion | 2,394,486 |

As shown in Table 20, also for a magnitude of the amount of luminescence, intensity of a signal increased in the order of amnion, placenta, decidua, and chorion.

This was similar to the results shown in FIG. 2.

From this result, the produced anti-15-PGDH monoclonal antibody for a solid phase and for labeling were shown to be a useful pair as an antibody to be supported on a solid-phase carrier and as a raw material of a labeled anti-15-PGDH antibody used in the testing method of the present invention.

INDUSTRIAL APPLICABILITY

The present invention is useful for clinical laboratory testing, particularly prediction of success or failure of induction of labor, prediction of whether or not preterm birth is likely to occur, and prediction of whether or not preterm labor is likely to occur.

SEQUENCE LISTING

SY631WO_1661757067647.xml

**Claims**

1. A testing method using an expression level of 15-PGDH as an index for success or failure of induction of labor, the testing method comprising:

   a step of detecting the expression level of 15-PGDH in a specimen,
   the specimen being a vaginal secretion.

2. The testing method according to claim 1, further comprising an evaluation step of evaluating a magnitude of the expression level of 15-PGDH in the specimen by comparing a signal of the expression level of 15-PGDH detected with an index of success or failure of induction of labor related to the signal.

3. The testing method according to claim 2,
   wherein the signal of the expression level of 15-PGDH is a value obtained by dividing the expression level of 15-PGDH by an expression level of prostaglandin.

4. A testing method using an expression level of 15-PGDH as an index for preterm birth, the testing method comprising:

   a step of detecting the expression level of 15-PGDH in a specimen,
   the specimen being a vaginal secretion.

5. The testing method according to claim 4, further comprising an evaluation step of evaluating a magnitude of the expression level of 15-PGDH in the specimen by comparing a signal of the expression level of 15-PGDH detected with an index of preterm birth related to the signal.

6. The testing method according to claim 5,
   wherein the signal of the expression level of 15-PGDH is a value obtained by dividing the expression level of 15-PGDH by an expression level of prostaglandin.

7. A testing method using an expression level of 15-PGDH as an index for preterm labor, the testing method comprising:

   a step of detecting the expression level of 15-PGDH in a specimen,
   the specimen being a vaginal secretion.

8. The testing method according to claim 7, further comprising an evaluation step of evaluating a magnitude of the expression level of 15-PGDH in the specimen by comparing a signal of the expression level of 15-PGDH detected with an index of preterm labor related to the signal.

9. The testing method according to claim 8,
wherein the signal of the expression level of 15-PGDH is a value obtained by dividing the expression level of 15-PGDH by an expression level of prostaglandin.

10. The testing method according to any one of claims 1 to 9,
wherein the vaginal secretion is a secretion from an epithelium of a cervix and/or a vaginal squamous epithelium.

11. The testing method according to any one of claims 1 to 10,
wherein the vaginal secretion is a secretion derived from a primiparous woman.

12. The testing method according to any one of claims 1 to 11,
wherein the step of detecting is detection by immunological measurement.

13. The testing method according to claim 12, further comprising a step of quantifying the 15-PGDH.

14. The testing method according to any one of claims 1 to 11,
wherein the step of detecting is detection by gene-related testing.

15. The testing method according to claim 14, further comprising a step of quantifying mRNA of the 15-PGDH.

16. A testing reagent for prediction of success or failure of induction of labor, the testing reagent comprising an anti-15-PGDH antibody or a primer for 15-PGDH.

17. The testing reagent according to claim 16, which is used in the testing method according to any one of claims 1 to 3 and 10 to 15.

18. A testing reagent for prediction of preterm birth, the testing reagent comprising an anti-15-PGDH antibody or a primer for 15-PGDH.

19. The testing reagent according to claim 18, which is used in the testing method according to any one of claims 4 to 6 and 10 to 15.

20. A testing reagent for prediction of preterm labor, the testing reagent comprising an anti-15-PGDH antibody or a primer for 15-PGDH.

21. The testing reagent according to claim 20, which is used in the testing method according to any one of claims 7 to 15.

FIG.1A

FIG.1B

FIG.2

FIG.3A

mRNA of 15-PGDH in vaginal secretion

FIG.3B

mRNA of 15-PGDH in vaginal secretion

FIG.4A

mRNA of 15-PGDH in vaginal secretion

FIG.4B

mRNA of 15-PGDH in vaginal secretion

FIG.5

Expression level of 15-PGDH in vaginal secretion

FIG.6A

Bishop score

FIG.6B

**Bishop score**

FIG.7A

Bishop score

FIG.7B

Bishop score

FIG.8

15-PGDH in vaginal secretion

FIG.9

15-PGDH in vaginal secretion

FIG.10

15-PGDH in vaginal secretion

FIG.11

15-PGDH in vaginal secretion

<div align="center">

**INTERNATIONAL SEARCH REPORT**

</div>

| | International application No. |
|---|---|
| | **PCT/JP2022/032442** |

| **A.** | **CLASSIFICATION OF SUBJECT MATTER** |
|---|---|

*G01N 33/573*(2006.01)i; *C07K 16/40*(2006.01)i; *C12Q 1/68*(2018.01)i; *C12Q 1/6876*(2018.01)i
FI:    G01N33/573 A; C12Q1/6876 Z; C07K16/40; C12Q1/68 ZNA

According to International Patent Classification (IPC) or to both national classification and IPC

| **B.** | **FIELDS SEARCHED** |
|---|---|

Minimum documentation searched (classification system followed by classification symbols)

G01N33/573; C07K16/40; C12Q1/68; C12Q1/6876

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| **C.** | **DOCUMENTS CONSIDERED TO BE RELEVANT** | |
|---|---|---|

| Category* | Citation of document, with indication, where appropriate, of the relevant passages | Relevant to claim No. |
|---|---|---|
| A | WO 2019/010482 A1 (CASE WESTERN RESERVE UNIVERSITY) 10 January 2019 (2019-01-10) entire text, all drawings, in particular, see claims, paragraphs [0004], [0006], [0017]-[0032], [0098]-[0107], etc. | 1-15, 17, 19, 21 |
| X | | 16, 18, 20 |
| A | US 2013/0280241 A1 (THE BRIGHAM AND WOMEN'S HOSPITAL, INC.) 24 October 2013 (2013-10-24) entire text, all drawings, in particular, see paragraphs [0019]-[0020], [0074]-[0075], [0087]-[0088], etc. | 1-21 |
| A | JP 8-506658 A (ADEZA BIOMEDICAL CORPORATION) 16 July 1996 (1996-07-16) see entire text, etc. | 1-21 |
| A | JP 2008-514900 A (ADEZA BIOMEDICAL CORPORATION) 08 May 2008 (2008-05-08) see entire text, etc. | 1-21 |
| A | US 2019/0369109 A1 (SERA PROGNOSTICS, INC.) 05 December 2019 (2019-12-05) see entire text, etc. | 1-21 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **27 October 2022** | **08 November 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

<div style="text-align: center;">

**INTERNATIONAL SEARCH REPORT**

</div>

| | International application No. |
|---|---|
| | **PCT/JP2022/032442** |

| | |
|---|---|
| **Box No. I** | **Nucleotide and/or amino acid sequence(s) (Continuation of item 1.c of the first sheet)** |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    The "form of an Annex C/ST.25 text file" is replaced with the "form of ST.26."

**INTERNATIONAL SEARCH REPORT**
Information on patent family members

| International application No. |
| --- |
| **PCT/JP2022/032442** |

| Patent document cited in search report | | | Publication date (day/month/year) | Patent family member(s) | | | Publication date (day/month/year) |
| --- | --- | --- | --- | --- | --- | --- | --- |
| WO | 2019/010482 | A1 | 10 January 2019 | (Family: none) | | | |
| US | 2013/0280241 | A1 | 24 October 2013 | US entire text, all drawings, in particular, see paragraphs [0026]-[0027], etc. | 2010/0284989 | A1 | |
| | | | | WO | 2010/129712 | A1 | |
| JP | 8-506658 | A | 16 July 1996 | WO see entire text, etc. | 1994/017405 | A1 | |
| | | | | EP | 680607 | A1 | |
| | | | | JP | 2004-125807 | A | |
| | | | | JP | 2005-326427 | A | |
| JP | 2008-514900 | A | 08 May 2008 | US see entire text, etc. | 2006/0024722 | A1 | |
| | | | | US | 2006/0024723 | A1 | |
| | | | | US | 2006/0024724 | A1 | |
| | | | | US | 2006/0024725 | A1 | |
| | | | | US | 2006/0024757 | A1 | |
| | | | | US | 2012/0040356 | A1 | |
| | | | | US | 2013/0171672 | A1 | |
| | | | | US | 2015/0044708 | A1 | |
| | | | | WO | 2006/026020 | A2 | |
| | | | | EP | 2299275 | A2 | |
| | | | | JP | 2011-39068 | A | |
| US | 2019/0369109 | A1 | 05 December 2019 | US see entire text, etc. | 2018/0143202 | A1 | |
| | | | | WO | 2018/027160 | A1 | |
| | | | | EP | 3494232 | A1 | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013019889 A

**Non-patent literature cited in the description**

- *Obstetrics and Gynecology,* 1964, vol. 24 (2), 266-268
- *Obstetrics and Gynecology,* 2006, vol. 107 (2), 227-233
- *The New England Journal of Medicine,* 1991, vol. 325 (10), 669-674
- *Acta Obsret Cynecol Scand,* 1991, vol. 70 (1), 29-34
- *Eur. J immunol,* 1976, vol. 6, 511
- Enzyme Immunoassay. Igaku-Shoin Ltd, 15 May 1987
- **S. YOSHITAKE ; M. IMAGAWA ; E. ISHIKAWA et al.** *J. Biochem,* 1982, vol. 92, 1413-1424